# EUROPEAN PATENT APPLICATION

(11) **EP 2 386 631 A1**
(43) Date of publication of application: **16.11.2011**
(21) Application number: 11003882.5
(22) Date of filing: 11.05.2011
(51) Int. Cl.: C12N 9/20, C12Q 1/00

(54) **Purified lipase, methods of modification and applications**

(30) Priority: 16.05.2010 MY 1070026
(71) Applicant: Universiti Putra Malaysia, Selangor (MY)
(72) Inventor: Raja Noor, Zaliha Abd. Rahman, 43400 UPM, Serdang, Selangor (MY); Abu Bakar, Salleh, 43400 UPM, Serdang, Selangor (MY); Basri, Mahiran, 43400 UPM, Serdang, Selangor (MY); Azmiza Syawani, Jasni, 43400 UPM, Serdang, Selangor (MY)
(74) Representative: Krauss, Jan

(57) **Abstract**

The present invention relates to the modification of 205y lipase sequence (removal of signal peptide and transmembrane domain) by polymerase chain reaction technique. Also, theis invention discolses crude 205y lipase (-SP/TM) that was successfully purified to homogeneity. Indeed, the removal of signal peptide and transmembrane domain from 205y lipase provides higher activity with improvements on pH and temperature optimum as well as tolerance towards organic solvents

SEQUENCE LISTING

<110>  Universiti Putra Malaysia

<120>  PURIFIED LIPASE, METHODS OF MODIFICATION AND APPLICATIONS

<130>  FB24934

<150>  MY2010700026
<151>  2010-05-16

<160>  6

<170>  PatentIn version 3.5

<210>  1
<211>  1191
<212>  DNA
<213>  Bacillus sphaericus

<400>  1
atgaatcaga taacaaattg gcgacctaga ggttttcttc agtggctact cacaatcctc      60

tctgtgatcg tatgttttat aatcattata gtggcatact atatttttaa tccaagtaat     120

atggataaaa taatgagtct ccttgcatgg gggacttcta tatttccgat tatgctagta     180

gttacagcat tcataataat cgttttacta gctctttctt tttggaagaa aacattcatt     240

gctcttacag tgttatttcc tattttgttg ttattaatgt ttttaactgt gcagccaatt     300

agtactatga aaagctatgc taaaagcgag aatgtatcgg ttgctcttag ctcacatttt     360

ttctataacc aaaatatctc aacaaaacct agtgtagatg tggtgtatgg gaaaactacg     420

gacggcattg aactaaagct tgatgtgtgg ccagcaaaga aaaaatcaga ggatgtttta     480

acacctgtca ttgttcaagt gcatgggggt ggttgggtaa gtggagataa aggccaagtg     540

caagactgga atcagtggat gaatgatcaa gggtacactg tttttgatgt ccaataccgt     600

atgcctcctg tagcaggctg gaaagatgaa gtgggggatg ttaaatcagc gattggttgg     660

attgtgcaac atgcagatac gtataagatt gatccgaaca gaatcatttt aatgggagag     720

tctgctgggg gaaatctagc tatgcttgct gcctatagtt tgggagataa gcatttaccg     780

ccatcaacgg atgttccgga tgtaccgatc aaggcagtga ttaacatgta tgggccgtca     840

gatatgactg cgttttacaa gaacaatcca agtaagcgtt atgtccaaga tgttctggat     900

cagtacattg gaggttcacc atccgattac cctgcgcgtt ataagaagct ttcccctata     960

agctatatcc aggaacatac gccaccaact attatgtttt tagggacggg tgatcgtatt    1020

gttcctgtag agcaggcaaa cgtattggat gacaaactga cgacaagcgg tgtagcgcat    1080

gaactttatc ttcttccaaa ggtcgatcat ggctttgatg caaatccagg tagtttgagc    1140

acgcaatttg ccaaggagaa agtaaaagca tttcttcaaa aatataataa a             1191

<210>  2
<211>  1095
<212>  DNA
<213>  Bacillus sphaericus

<400>  2

tactatattt ttaatccaag taatatggat aaaataatga gtctccttgc atgggggact 60
tctatatttc cgattatgct agtagttaca gcattcataa taatcgtttt actagctctt 120
tctttttgga agaaaacatt cattgctctt acagtgttat ttcctatttt gttgttatta 180
atgtttttaa ctgtgcagcc aattagtact atgaaaagct atgctaaaag cgagaatgta 240
tcggttgctc ttagctcaca ttttttctat aaccaaaata tctcaacaaa acctagtgta 300
gatgtggtgt atgggaaaac tacggacggc attgaactaa agcttgatgt gtggccagca 360
aagaaaaaat cagaggatgt tttaacacct gtcattgttc aagtgcatgg gggtggttgg 420
gtaagtggag ataaaggcca agtgcaagac tggaatcagt ggatgaatga tcaagggtac 480
actgtttttg atgtccaata ccgtatgcct cctgtagcag gctggaaaga tgaagtgggg 540
gatgttaaat cagcgattgg ttggattgtg caacatgcag atacgtataa gattgatccg 600
aacagaatca ttttaatggg agagtctgct gggggaaatc tagctatgct tgctgcctat 660
agtttgggag ataagcattt accgccatca acggatgttc cggatgtacc gatcaaggca 720
gtgattaaca tgtatgggcc gtcagatatg actgcgtttt acaagaacaa tccaagtaag 780
cgttatgtcc aagatgttct ggatcagtac attggaggtt caccatccga ttaccctgcg 840
cgttataaga agctttcccc tataagctat atccaggaac atacgccacc aactattatg 900
tttttaggga cgggtgatcg tattgttcct gtagagcagg caaacgtatt ggatgacaaa 960
ctgacgacaa gcggtgtagc gcatgaactt tatcttcttc caaaggtcga tcatggcttt 1020
gatgcaaatc caggtagttt gagcacgcaa tttgccaagg agaaagtaaa agcatttctt 1080
caaaaatata ataaa 1095

<210> 3
<211> 900
<212> DNA
<213> Bacillus sphaericus

<400> 3
cagccaatta gtactatgaa aagctatgct aaaagcgaga atgtatcggt tgctcttagc 60
tcacattttt tctataacca aaatatctca acaaaaccta gtgtagatgt ggtgtatggg 120
aaaactacgg acggcattga actaaagctt gatgtgtggc cagcaaagaa aaaatcagag 180
gatgttttaa cacctgtcat tgttcaagtg catgggggtg gttgggtaag tggagataaa 240
ggccaagtgc aagactggaa tcagtggatg aatgatcaag ggtacactgt ttttgatgtc 300
caataccgta tgcctcctgt agcaggctgg aaagatgaag tgggggatgt taaatcagcg 360
attggttgga ttgtgcaaca tgcagatacg tataagattg atccgaacag aatcatttta 420
atgggagagt ctgctggggg aaatctagct atgcttgctg cctatagttt gggagataag 480
catttaccgc catcaacgga tgttccggat gtaccgatca aggcagtgat taacatgtat 540
gggccgtcag atatgactgc gttttacaag aacaatccaa gtaagcgtta tgtccaagat 600
gttctggatc agtacattgg aggttcacca tccgattacc ctgcgcgtta taagaagctt 660
tcccctataa gctatatcca ggaacatacg ccaccaacta ttatgttttt agggacgggt 720

gatcgtattg ttcctgtaga gcaggcaaac gtattggatg acaaactgac gacaagcggt 780

gtagcgcatg aactttatct tcttccaaag gtcgatcatg gctttgatgc aaatccaggt 840

agtttgagca cgcaatttgc caaggagaaa gtaaaagcat ttcttcaaaa atataataaa 900

<210> 4
<211> 21
<212> DNA
<213> artificial

<220>
<223> Primer

<400> 4
atgaatcaga taacaaattg g 21

<210> 5
<211> 24
<212> DNA
<213> artificial

<220>
<223> Primer

<400> 5
tactatattt ttaatccaag taat 24

<210> 6
<211> 24
<212> DNA
<213> artificial

<220>
<223> Primer

<400> 6
cagccaatta gtactatgaa aagc 24

## Description

### FIELD OF INVENTION

The present invention relates to a modified lipase sequence and characteristics of the modified lipase. More particularly, the lipase is obtained from *Bacillus sphaericus* strain 205y.

### BACKGROUND OF INVENTION

Microorganisms and higher eukaryotes produce lipases, however only microbial lipases are commercially significant. Lipase-producing microorganisms include bacteria, fungi, yeasts, and actinomycetes. In eukaryotes, lipases are involved in various stages of lipid metabolism including fat digestion, absorption, reconstitution, and lipoprotein metabolism. In plants, lipases are found in energy reserve tissues. Lipases are an excellent alternative to classical organic techniques in the selective transformation of complex molecules.

Bacterial lipases have been isolated from various genera and species including *Staphylococcus* (Gotz *et al.*, 1998), *Pseudomonas* (Lee *et al.*, 1993) as well as *Bacillus* (Lee *et al.*, 1999; Schmidt-Dannert *et al.*, 1994). Lipases from microbial show versatility in terms of their optimum pH and temperature, molecular weight as well as substrate specificity.

Lipases are one of the important mixtures in detergents available for fabrics cleaning as well as household laundering. The use of lipases in dairy industry, mainly for flavor development are well established (Yadav and Devi, 2004; Kanwar and Goswami, 2002; Xu *et al.*, 2002). The free fatty acids produced by the action of lipases on milk fat endowed many dairy products. Addition of lipases that release short chain (mainly C4 and C6) fatty acids normally lead to the development of a sharp and tangy flavor, while long chain (C12 and C14) fatty acids tend to impart a soapy taste to the product.

Free fatty acids produced also take part in simple chemical reactions, as well as being converted to other by-products such as acetoacetate, methyl ketones, lactones and beta-keto acids, for which those by-products enhance the flavor of the dairy products.

The usefulness of lipases in waste treatment is well recognized. Here, lipases are used in activated sludge and other aerobic waste processes, in which the thin layer of fats must be continuously removed from the surface of aerated tanks to permit oxygen transport. As lipases in sewage treatment are effective in breaking down solids, thus blockage of fats in waste systems can be prevented (Jaeger and Reetz, 1998).

Enzyme molecules in aqueous solutions have hydrophilic and hydrophobic domains. The hydrophilic domain tends to have a direct contact with water while hydrophobic domain, on the other hand, folds inside the molecules. Upon the presence of organic solvent, the polarity of the medium surrounding the enzyme molecules will reduce (Ogino and Ishikawa, 2000). As a result, the hydrophobic domain is apt to disperse, thus resulting in the unfolding of the molecules. Therefore, organic solvents such as toluene, ethanol and dimethyl sulfoxide (DMSO) have been anticipated to cause rapid denaturation, inactivation as well as severe distortion of the enzyme structure upon direct contact (Ogino and Ishikawa, 2000).

Hence, all this while, many attempts have been carried out in order to screen for both the solvent-tolerant microorganism and the organic solvent-stable enzyme secreted by these microbes that could withstand such a harsh environment (Inoue and Horikoshi, 1989). So far, most of the reported species that represent both characteristics are *Pseudomonas* strains (Ogino *et al.*, 2000; Kobayashi *et al.*, 1999; Cruden *et al.*, 1992; Inoue *et al.*, 1991) as well as *Staphylococcus* strains (Simons *et al.*, 1997).

One of the more serious drawbacks for the use of enzymes in aqueous organic synthesis is the poor water solubility or organic compounds of more than 4 carbon atoms. Water is also a poor solvent for most applications in industrial chemistry, since many organic compounds are unstable in aqueous solution. The use of organic solvents presents several advantages, including: a) easier recovery of products with high yields; b) the possibility of using non-polar substrates; c) organic solvents avoid many side reactions.

*Bacillus sphaericus* 205y belongs to the order of *Bacillales*, is typically rods and gram-positive spore-forming bacterium. The growth temperature of the *Bacillus sphaericus* 205y cells is up to 55°C with optimum growth lies near 37°C. This bacterium was also reported to grow at high concentration of solvents up to 75% (v/v) (Chin, 2003).

In the present invention, the activity of a wild type organic solvent stable lipase secreted by *Bacillus sphaericus* 205y was successfully investigated. Here, the enzyme activity was enhanced by 2.9- and 3.5-fold upon the presence of *p*-xylene and *n*-hexane, respectively. In contrast, approximately 80% of lipase activity was lost in the presence of dimethyl sulfoxide (DMSO) while complete deactivation reported for acetonitrile (Chin, 2003).

The present invention responds specifically to the long-felt need heretofore unmet by the prior art, and especially with a view to overcoming the objective of the present invention which relates to the modification of 205y lipase sequence (removal of signal peptide and transmembrane domain) by polymerase chain reaction technique.

Yet another objective of the present invention provides crude 205y lipase (-SP/TM) that was successfully purified to homogeneity. Indeed, the removal of signal peptide and transmembrane domain from 205y lipase provides higher activity with improvements on pH and temperature optimum as well as tolerance towards organic solvents. This is in comparison with ORF as revealed by Sulong *et al.* (2006). The above justification contributes to catalytic activity of this enzyme as previously hypothesized.

### Object to be solved by the present invention

The present application relates to an improved structure using computational approach, therefore further confirmation on the effect of signal peptide and transmembrane domain removal to 205y lipase. Also, using crystallization and x-ray diffraction analysis is recommended to be useful on 205y lipase.

Moreover, the present application notably provides the effect of removal of signal peptide and transmembrane domain on 205y lipase, wherein the 205y lipase secondary structure, with respect to the melting temperature (Tm) is analyse.

Also, the current invention shall provide a scale-up production of 205y lipase for industrial purposes, whereby the 205y lipase (-SP/TM) can be expressed in yeast expression system, followed by fermentation using a bio-reactor.

### SUMMARY OF THE INVENTION

The present invention discloses a novel organic solvent tolerant lipase gene obtained from a biologically pure culture of *Bacillus sphaericus.* The lipase gene includes 3 lipase gene(s) of SEQ ID NO 1, SEQ ID NO 2 and SEQ ID NO 3 (the lipase gene is 205y lipase)

Moreover, the present invention provides a method of obtaining organic solvent 205y lipase genes, the method provides the means of using primers to amplify the gene(s) by polymerase chain reaction (PCR) technique.The primers to amplify the genes, includes:
a) 5'-ATG AAT CAG ATA ACA AAT TGG-3'(SEQ ID NO4) (amplify the 205y lipase gene with an open reading frame) (amplification size of 1191 base pair);
b) 5'-TAC TAT ATT TTT AAT CCA AGT AAT-3'(SEQ ID NO5) (amplify the 205y lipase gene without a signal peptide)( amplification size of 1095 base pair);
c) 5'-CAG CCA ATT AGT ACT ATG AAA AGC-3' (SEQ ID NO6) (amplify the 205y lipase gene without a signal peptide and transmembrane domain)( amplification size of 900 base pair).

The present invention further relates to a purified 205y lipase without signal peptide and transmembrane domain is obtained from the preceding method. The purified 205y lipase without signal peptide and transmembrane domain including the steps harvesting recombinant *E. coli* by centrifugation at 10,000 revolution per min (rpm), for 10 min at 4°C; obtaining pellet and dissolving the pellet in tris-HCl buffer (10mM) at pH between 7.0-7.3; performing sonication of the pellet and obtaining cell lysate; mixing the cell lysate with Bio-Lyte ampholyte (40% w/v); obtaining a mixture of cell lysate with ampholyte and loading the mixture of cell lysate with ampholyte into a protein separation chamber; obtaining purified fractions of protein.

Additionally, the present invention describes a method of characterizing purified 205y lipase without a signal peptide and transmembrane domain, wherein the method includes the steps analysing temperature on lipase activity and stability; analysing pH on lipase activity and stability using buffers, wherein the buffers comprises acetate buffer (pH 4.0-6.0), potassium phosphate buffer (pH 6.0-8.0), tris-HCl buffer (pH 8.0-9.0), glycine-NaOH buffer (pH 9.0-11.0) and Na₂HPO₄/NaOH buffer; analysing organic solvents on lipase activity, wherein the organic solvents comprises DMSO, methanol, ethanol, acetonitrile, *n*-decane, hexadecane, *n*-butanol, toluene, *p*-xylene, 1-decanol and *n-*dodecane; analysing metal ion on lipase activity, wherein the metal ions includes K⁺, Mg²⁺ Ca²⁺, Na⁺, Mn²⁺, Fe²⁺, Cu²⁺ and Zn²⁺; analysing surfactants on lipase activity; wherein the surfactants comprises Tween 20, Tween 40, Tween 60, Tween 80, Triton X-100, SDS and SLS; analysing inhibitors on lipase activity; wherein the inhibitors comprises dithiothreitol (DTT), β-mercaptoethanol, phenylmethylsulfonyl fluoride (PMSF), ethylenediaminetetraacetic acid (EDTA) and pepstatin A; analysing substrate specificity of the 205y lipase, wherein the substrate comprises triacylglycerols (triacetin, tributyrin, tricaprilyn, triolein) and natural oils (olive oil, soy bean oil, sun flower oil, rice bran oil, coconut oil and palm oil); determining positional specificity of the 205y lipase. The method of characterizing purified 205y lipase further comprising the steps of preparing a reaction mixture by mixing 100 µL of purified enzyme with 2.5 mL substrate [olive oil: phosphate buffer pH 7.0, 1:1, (v/v)] and 20 µL CaCl_{2;} heating the reaction mixture at temperature between 30°C and 70°C for 30 min; agitating the reaction mixture at 200 rpm; measuring lipase activity; allowing the reaction mixture to pre-incubate at temperature between 37°C and 60°C for 30 min; agitating the reaction mixture at 200 rpm at 60°C; and measuring lipase activity. Moreover, the method of characterizing purified 205y lipase further includes preparing a reaction mixture by mixing 100 µL of purified enzyme with 2.5 mL substrate [olive oil: the buffers between pH 4 and 12, 1:1, (v/v)] and 20 µL CaCl_{2;} heating the reaction mixture at temperature between 30°C and 70°C for at least 30 min; agitating the reaction mixture at 200 rpm; measuring lipase activity; allowing the reaction mixture to pre-incubate with buffers at temperature at 37°C between pH 4 and 12 for at least 30 min; agitating the reaction mixture from step (e) at 200 rpm at 60°C; and measuring lipase activity.

The purified 205y lipase without a signal peptide and transmembrane domain having the means of providing an activity between 55°C and 65°C.The purified 205y lipase without a signal peptide and transmembrane domain having the means of providing stability at pH 5 and 10. The purified 205y lipase without a signal peptide and transmembrane domain having the means of providing stability with organic solvents such as DMSO, methanol, ethanol, acetonitrile, n-decane, hexadecane.

The purified 205y lipase without a signal peptide and transmembrane domain having the means of providing an activity in the presence of K⁺, Mg²⁺ and Ca²⁺. The purified 205y lipase without a signal peptide and transmembrane domain having the means of providing an activity in the presence of Tween 20, Tween 40, Tween 60, Tween 80, Triton X-100 and SDS. The purified 205y lipase without a signal peptide and transmembrane domain having the means of providing an activity in the presence hydrolyzing triglycerides and natural oils at position 1 and 3.

### BRIEF DESCRIPTION OF DRAWINGS

The accompanied drawings constitute part of this specification and include an exemplary or preferred embodiment of the invention, which may be embodied in various forms. It should be understood, however, the disclosed preferred embodiments are merely exemplary of the invention. Therefore, the figures disclosed herein are not to be interpreted as limiting, but merely as the basis for the claims and for teaching one skilled in the art of the invention
Figure 1 shows PCR Amplification of 205y Lipase Genes using 3 Forward Primers. Lane 1: Open reading frame of 205y lipase (ORF); Lane 2: 205y lipase without signal peptide (-SP); Lane 3: 205y lipase without signal peptide and transmembrane domain (-SP/TM); M: 1kb DNA Ladder.
Figure 2 shows production of Clearing Zones on LB/Tributyrin Agar by *E. coli* TOP10 Harboring pBAD/ -SP/TM Gene.
Figure 3 shows circular Form of Recombinant Plasmids Harboring 205y Lipase Genes. M: 1kb DNA Ladder, Lanes 1 and 2: pBAD/ORF; Lanes 3 and 4: pBAD/-SP and Lane 5 to 7: pBAD/-SP/TM.
Figure 4 shows analysis of Recombinant Genes using 205y Lipase Forward and 205y Lipase Reverse Primers. M: 1kb DNA Ladder, Lane 1 and 2: pBAD/ORF; Lane 3 and 4: pBAD/-SP and Lane 5 to 7: pBAD/-SP/TM
Figure 5 shows analysis of Recombinant Genes using 205y Lipase Forward and pBAD Reverse Primers. M: 1kb DNA Ladder, Lane 1 and 2: pBAD/ORF; Lane 3 and 4: pBAD/- SP and Lane 5 to 7: pBAD/-SP/TM.
Figure 6 shows multiple Sequence Alignment of 205y Lipase (ORF, -SP and SP/TM) using Clustal W Algorithm. The asterisks indicate consensus of the three sequences.
Figure 7 shows effect of Inducer's Concentration on Expression of 205y Lipase (-SP/TM). *E. coli* TOP10 harboring recombinant plasmid pBAD/-SP/TM was induced with different concentrations of inducer; 0.00002%, 0.0002%, 0.002% and 0.02% at *A*₆₀₀ₙₘ ∼0.5 for 24 h.
Figure 8 shows effect of Induction Time on Expression of 205y Lipase (-SP/TM). *E. coli* TOP10 harboring recombinant plasmid pBAD/-SP/TM was induced with 0.0002% L-arabinose at *A*₆₀₀ₙₘ ∼0.5 and was cultivated up to 20 h.
Figure 9 shows effect of Induction *A*₆₀₀ₙₘ on Expression of 205y Lipase (-SP/TM) by *E. coli. E. coli* TOP10 harboring recombinant plasmid pBAD/ -SP/TM was induced with 0.0002% L-arabinose and was cultivated for 12 h.
Figure 10 shows purification Profile of 205y Lipase (-SP/TM) using pH Range of 3-10. Highest lipase activity was detected at fraction 12 (6.5 U/mL). Fractions with highest lipase activity (fraction 10-12) were then subjected for further analysis using SDS-PAGE and activity staining.
Figure 11 shows SDS-PAGE (12%) Analysis of Purified 205y Lipase (-SP/TM). M: Standard protein markers were β-galactosidase (116.0 kDa), bovine serum albumin (66.2 kDa), ovalbumin (45.0 kDa), lactate dehydrogenase (35.0 kDa), restriction endonuclease *Bsp* 981 (25.0 kDa), β-lactoglobulin (18.4 kDa) and lysozyme (14.4 kDa); Lane 1-3: Purified 205y lipase (-SP/TM); C: Crude 205y lipase (-SP/TM). Arrow indicates the purified 205y lipase (-SP/TM) with the size of 30 kDa.
Figure 12 shows staining Gel of Purified 205y Lipase (-SP/TM). The proteins were electrophoresed and subsequently transferred onto LB/tributyrin agar and incubated at 37°C. Lane 1-3: Purified 205y lipase (-SP/TM); M: Prestained protein molecular weight marker. Arrow indicates the purified lipase with the size of 30 kDa.
Figure 13 shows temperature Profile of 205y Lipase (-SP/TM). The 205y lipase (-SP/TM) was assayed with olive oil as substrate at temperature ranging from 30°C to 70°C at pH 7.0 for 30 min. Relative activities are mean values ± standard deviations of three determinations (n=3).
Figure 14 shows thermostability Profile of 205y Lipase (-SP/TM). The 205y lipase (-SP/TM) was incubated at different temperatures in phosphate buffer (pH 7.0) and the activity was assayed at respective time intervals. The symbols used are: (●) 37°C; (◆) 50°C; (▲) 55°C and (■) 60°C. Relative activities are mean values ± standard deviations of three determinations (n=3).
Figure 15 shows pH Profile of 205y Lipase (-SP/TM). The 205y lipase (-SP/TM) was assayed at various pH ranging from pH 4.0 to pH 12.0 by spectrophotometric method. The symbols used are: (●) acetate buffer; (◆) pottassium phosphate buffer; (▲) Tris-HCl buffer; (■) glycine buffer and (x) Na₂HPO₄/NaOH buffer.
Figure 16 shows pH Stability of 205y Lipase (-SP/TM). The 205y lipase (-SP/TM) was incubated at 37°C for 30 min at various pH (1:1, v/v) ranging from pH 4.0 to pH 12.0 and assayed using spectrophotometric method. The symbols used are: (●) acetate buffer; (◆) pottassium phosphate buffer; (▲) Tris-HCl buffer; (■) glycine buffer and (x) Na₂HPO₄/NaOH buffer.
Figure 17 shows stability of 205y Lipase (-SP/TM) Towards Organic Solvents. Relative activities are mean values ± standard deviations of three determinations (n=3).
Figure 18 shows effect of metal ions on 205y Lipase (-SP/TM) Activity. 205y lipase (-SP/TM) was incubated at 37°C for 30 min with different metal ions at 1 mM (□) and 10 mM (□) prior to lipase assay. Relative activities are mean values ± standard deviations of three determinations (n=3).
Figure 19 shows substrate Specificity of 205y Lipase (-SP/TM) Towards Different Carbon Chain Length of Triglycerides and Natural Oils. Relative activities of the enzyme against substrates are shown as values relative to that against olive oil.
Figure 20 shows thin Layer Chromatography (TLC) Analysis of Hydrolysis Products After Incubation of 205y Lipase (-SP/TM) on Triolein as Substrate at 37°C for 30 min. Lane 1: Mixture of standard marker; Lane 2: 1,2 (1,3)-diolein; Lane 3: Oleic acid; Lane 4: Triolein; Lane 5: 205y lipase (-SP/TM) (0 h); Lane 6: 205y lipase (-SP/TM) (0.5 h); Lane 7: Lipozyme (0 h); Lane 8: Lipozyme (0.5 h); Lane 9: Novozyme (0 h) and Lane 10: Novozyme (0.5 h). Spots were developed by iodine vapor.

### SEQUENCE LISTING

### SEQ ID NO 1

### Full ORF 205y Lipase Sequence

ATGAATCAGATAACAAATTGGCGACCTAGAGGTTTTCTTCAGTGGCTACT
CACAATCCTCTCTGTGATCGTATGTTTTATAATCATTATAGTGGCATACT
ATATTTTTAATCCAAGTAATATGGATAAAATAATGAGTCTCCTTGCATGG
GGGACTTCTATATTTCCGATTATGCTAGTAGTTACAGCATTCATAATAAT
CGTTTTACTAGCTCTTTCTTTTTGGAAGAAAACATTCATTGCTCTTACAG
TGTTATTTCCTATTTTGTTGTTATTAATGTTTTTAACTGTGCAGCCAATT
AGTACTATGAAAAGCTATGCTAAAAGCGAGAATGTATCGGTTGCTCTTAG
CTCACATTTTTTCTATAACCAAAATATCTCAACAAAACCTAGTGTAGATG
TGGTGTATGGGAAAACTACGGACGGCATTGAACTAAAGCTTGATGTGTGG
CCAGCAAAGAAAAAATCAGAGGATGTTTTAACACCTGTCATTGTTCAAGT
GCATGGGGGTGGTTGGGTAAGTGGAGATAAAGGCCAAGTGCAAGACTGGA
ATCAGTGGATGAATGATCAAGGGTACACTGTTTTTGATGTCCAATACCGT
ATGCCTCCTGTAGCAGGCTGGAAAGATGAAGTGGGGGATGTTAAATCAGC
GATTGGTTGGATTGTGCAACATGCAGATACGTATAAGATTGATCCGAACA
GAATCATTTTAATGGGAGAGTCTGCTGGGGGAAATCTAGCTATGCTTGCT
GCCTATAGTTTGGGAGATAAGCATTTACCGCCATCAACGGATGTTCCGGA
TGTACCGATCAAGGCAGTGATTAACATGTATGGCCGTCAGATATGACTG
CGTTTTACAAGAACAATCCAAGTAAGCGTTATGTCCAAGATGTTCTGGAT
CAGTACATTGGAGGTTCACCATCCGATTACCCTGCGCGTTATAAGAAGCT
TTCCCCTATAAGCTATATCCAGGAACATACGCCACCAACTATTATGTTTT
TAGGGACGGGTGATCGTATTGTTCCTGTAGAGCAGGCAAACGTATTGGAT
GACAAACTGACGACAAGCGGTGTAGCGCATGAACTTTATCTTCTTCCAAA
GGTCGATCATGGCTTTGATGCAAATCCAGGTAGTTTGAGCACGCAATTTG

CCAAGGAGAAAGTAAAAGCATTTCTTCAAAAATATAATAAA
10

### SEQ ID NO 2

### Sequence of 205y Lipase without Signal Peptide

---------------------------------------------TACT
ATATTTTTAATCCAAGTAATATGGATAAAATAATGAGTCTCCTTGCATGG
GGACTTCTATATTTCCGATTATGCTAGTAGTTACAGCATTCATAATAAT
CGTTTTACTAGCTCTTTCTTTTTGGAAGAAAACATTCATTGCTCTTACAG
TGTTATTTCCTATTTTGTTGTTATTAATGTTTTTAACTGTGCAGCCAATT
AGTACTATGAAAAGCTATGCTAAAAGCGAGAATGTATCGGTTGCTCTTAG
CTCACATTTTTTCTATAACCAAAATATCTCAACAAAACCTAGTGTAGATG
TGGTGTATGGGAAAACTACGGACGGCATTGAACTAAAGCTTGATGTGTGG
CCAGCAAAGAAAAAATCAGAGGATGTTTTAACACCTGTCATTGTTCAAGT
GCATGGGGGTGGTTGGGTAAGTGGAGATAAAGGCCAAGTGCAAGACTGGA
ATCAGTGGATGAATGATCAAGGGTACACTGTTTTTGATGTCCAATACCGT
ATGCCTCCTGTAGCAGGCTGGAAAGATGAAGTGGGGGATGTTAAATCAGC
GATTGGTTGGATTGTGCAACATGCAGATACGTATAAGATTGATCCGAACA
GAATCATTTTAATGGGAGAGTCTGCTGGGGGAAATCTAGCTATGCTTGCT
GCCTATAGTTTGGGAGATAAGCATTTACCGCCATCAACGGATGTTCCGGA
TGTACCGATCAAGGCAGTGATTAACATGTATGGCCGTCAGATATGACTG
CGTTTTACAAGAACAATCCAAGTAAGCGTTATGTCCAAGATGTTCTGGAT
CAGTACATTGGAGGTTCACCATCCGATTACCCTGCGCGTTATAAGAAGCT
TTCCCCTATAAGCTATATCCAGGAACATACGCCACCAACTATTATGTTTT
TAGGGACGGGTGATCGTATTGTTCCTGTAGAGCAGGCAAACGTATTGGAT
GACAAACTGACGACAAGCGGTGTAGCGCATGAACTTTATCTTCTTCCAAA
GGTCGATCATGGCTTTGATGCAAATCCAGGTAGTTTGAGCACGCAATTTG
CCAAGGAGAAAGTAAAAGCATTTCTTCAAAAATATAATAAA

### SEQ ID NO 3

### Sequence of 205y Lipase without Signal Peptide and Transmembrane

---------------------------------------CAGCCAATT
AGTACTATGAAAAGCTATGCTAAAAGCGAGAATGTATCGGTTGCTCTTAG
CTCACATTTTTCTATAACCAAAATATCTCAACAAAACCTAGTGTAGATG
TGGTGTATGGGAAAACTACGGACGGCATTGAACTAAAGCTTGATGTGTGG
CCAGCAAAGAAAAAATCAGAGGATGTTTTAACACCTGTCATTGTTCAAGT
GCATGGGGGTGGTTGGGTAAGTGGAGATAAAGGCCAAGTGCAAGACTGGA
ATCAGTGGATGAATGATCAAGGGTACACTGTTTTTGATGTCCAATACCGT
ATGCCTCCTGTAGCAGGCTGGAAAGATGAAGTGGGGGATGTTAAATCAGC
GATTGGTTGGATTGTGCAACATGCAGATACGTATAAGATTGATCCGAACA
GAATCATTTTAATGGGAGAGTCTGCTGGGGGAAATCTAGCTATGCTTGCT
GCCTATAGTTTGGGAGATAAGCATTTACCGCCATCAACGGATGTTCCGGA
TGTACCGATCAAGGCAGTGATTAACATGTATGGCCGTCAGATATGACTG
CGTTTTACAAGAACAATCCAAGTAAGCGTTATGTCCAAGATGTTCTGGAT
CAGTACATTGGAGGTTCACCATCCGATTACCCTGCGCGTTATAAGAAGCT
TTCCCCTATAAGCTATATCCAGGAACATACGCCACCAACTATTATGTTTT
TAGGGACGGGTGATCGTATTGTTCCTGTAGAGCAGGCAAACGTATTGGAT
GACAAACTGACGACAAGCGGTGTAGCGCATGAACTTTATCTTCTTCCAAA
GGTCGATCATGGCTTTGATGCAAATCCAGGTAGTTTGAGCACGCAATTTG
CCAAGGAGAAAGTAAAAGCATTTCTTCAAAAATATAATAAA

### DETAILED DESCRIPTION OF THE INVENTION

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described. All publications mentioned hereunder are incorporated herein by reference.

### Definitions

As used herein, "amplification reaction mixture" or "amplification mixture" refers to an aqueous solution comprising the various reagents used to amplify a target nucleic acid. These include but are by no means limited to enzymes, aqueous buffers, salts, target nucleic acid and nucleoside triphosphates.

As used herein, "isolated" or "substantially pure", when referring to protein, refers to those which have been purified away from other cellular components and/or contaminants by standard techniques, for example, column chromatography, CsCl banding, and alkaline/SDS treatment as well as other techniques well known in the art.

As used herein, "primer" refers to an oligonucleotide capable of acting as a point of initiation of DNA synthesis under conditions in which synthesis of a primer extension product complementary to a nucleic acid strand is initiated. One skilled in the art will understand that substantially corresponding primers of the invention can vary from the referred-to sequence and still hybridize to the same target nucleic acid sequence. This variation from the nucleic acid may be stated in terms of a percentage of identical bases within the sequence or the percentage of perfectly complementary bases between the probe and its target sequence.

Accordingly, the present invention provides modifications of 205y lipase (removal of signal peptide and transmembrane domain) by using polymerase chain reaction technique. The 205y lipase enzyme having the means of increasing lipase activity.

Unexpectedly, the inventors have noted with the removal of signal peptide and transmembrane domain from 205y lipase has yielded higher activity with improvements on pH and temperature optimum as well as tolerance towards organic solvents compared to ORF as revealed by Sulong *et al.* (2006). These results illustrated the least contribution of both segments to the catalytic activity of this enzyme as previously hypothesized.

### BEST MODE TO CARRY OUT THE INVENTION

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims. When a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. When the stated range includes one or both of the limits, ranges excluding either or both of those included limits are also included in the invention.

### EXAMPLES

### Bacterial Strain and Plasmids

*Escherichia coli* TOP10 harboring pUC19/205y lipase (Accession no: AF453713) (Rahman *et al.*, 2003) in 80% (v/v) glycerol stock was obtained from Lab 140, Department of Microbiology, Faculty of Biotechnology and Biomolecular Sciences, UPM. Characteristics of bacterial strain and plasmids used in this study are shown in **Table 1.**

**Table 1. Bacterial strain and plasmids**

| Bacterial strain/plasmids | Purpose | Genotype |
|---|---|---|
| *Escherichia coli* TOP10* | Cloning and propagation of plasmids | F *mcr*A Δ (*mrr*-*hsd*RMS-*mcr*BC) Φ80*lac*ZΔM15 Δ*lac*X74 *rec*A1 *araD139* Δ(*ara-leu*)7697 *gal*U *gal*K *rps*L (Str^{R}) *end*A1 *nupG* |
| pUC19 | Cloning vector | |
| pBAD | General expression vector | |

| | | |
|---|---|---|
| * pBAD TOPO^{®} TA Expression Kit, Invitrogen, USA | | |

### Plasmid DNA (pUC19/205y Lipase) Extraction

*E. coli* strain TOP10 harboring pUC19/205y lipase was streaked onto Luria Bertani (LB) agar containing 15.0 (g/L) agar, 10.0 (g/L) tryptone, 10.0 (g/L) NaCl, 10.0 (g/L) gum arabic and 5.0 (g/L) yeast extract (adjusted to pH 7.0) supplemented with 1% (v/v) tributyrin and ampicillin (50 µg/mL). The plate was incubated overnight at 37°C. Single colonies were then inoculated into LB broth which contains 10.0 (g/L) tryptone, 10.0 (g/L) NaCl and 5.0 (g/L) yeast extract (adjusted to pH 7.2) with 50 µg/mL ampicillin. The cultures were incubated at 37°C under shaking condition (200 rpm). Plasmid from the overnight culture was then extracted and purified using QIAprep^{®} Miniprep Spin Kit (Qiagen, Germany) and QIAquick^{®} Gel Extraction Kit (Qiagen, Germany), respectively according to manufacturer's instructions. The purified DNA sample was electrophoresed on 1% (w/v) agarose gel to confirm the presence of pUC19/205y lipase.

### Preparation of Stock Culture

The 24 h pure culture from a single colony on LB agar plates was stored in 1.0 mL aliquots of 80% (v/v) sterilized glycerol stock at -80°C. The stock should be restreaked onto LB agar supplemented with tributyrin (1% v/v) and ampicillin (50 µg/mL) prior use.

### Polymerase Chain Reaction (PCR) Amplification of 205y Lipase

Four primers were designed to amplify 205y lipase gene at different points (**Table 2**). The first forward (ORF) amplified the open reading frame of 205y lipase gene (Accession no: AF453713), the second forward (-SP) amplified 205y lipase gene without signal peptide whilst the third forward (-SP/TM) amplified 205y lipase gene without both, the signal peptide and transmembrane domain. Details on the position of primers designed were disclosed in **Appendix A.**

**Table 2. PCR primers for amplification of 205y lipase gene**

| Primer | Sequence | Expected size (bp) |
|---|---|---|
| ORF | 5'-ATG AAT CAG ATA ACA AAT TGG-3' | 1191 |
| -SP | 5'-TAC TAT ATT TTT AAT CCA AGT AAT-3' | 1095 |
| -SP/TM | 5'-CAG CCA ATT AGT ACT ATG AAA AGC-3' | 900 |
| Reverse | 5' ATT ATA TTT TTG AAG AAA-3' | - |

PCR was carried out in 100 µL reaction mixture containing 10X PCR buffer (10 µL), 25 mM MgCl₂ (8 µL), 10 pmol/µL of each forward and reverse primers (3 µL), 10 mM dNTPmix (2 µL), *Taq* DNA polymerase (2 U) and DNA template (pUC19/205y lipase) (3 µL). The total volume of PCR mixture was topped up to 100 µL using distilled water.

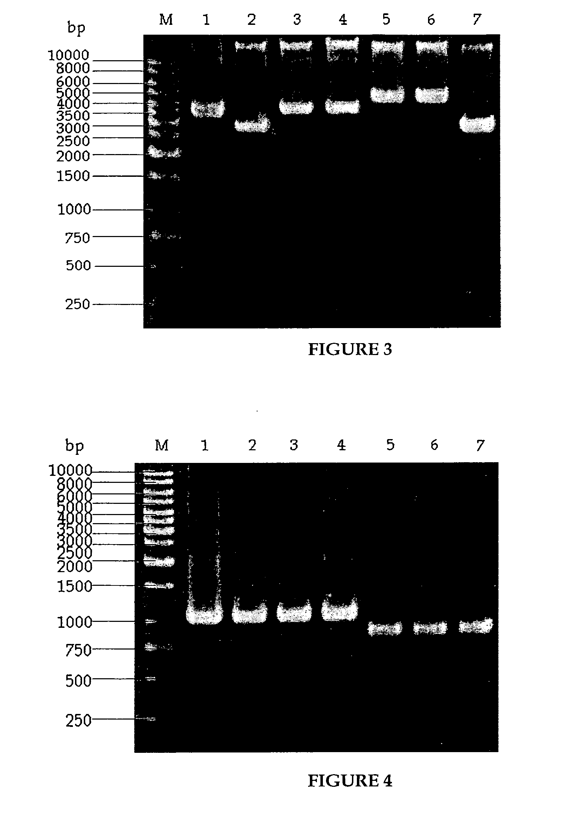

Amplification of the genes was carried out using gradient palm-cycler (Corbett Research, Australia) with the following conditions: initial denaturation step at 94°C for 4 min, 30 PCR cycles (94°C, 1 min; 46°C, 1 min and 72°C, 1 min), 1 cycle of 7 min at 72°C and preservation at 4°C for ∞. PCR products were then electrophoresed on 1% (w/v) agarose gel and visualized under UV radiation after staining with ethidium bromide (1 mg/mL) for 10-15 min. The amplified genes of 205y lipase (ORF, -SP and -SP/TM) were then purified using QIAquick^{®} Gel Extraction Kit (Qiagen, Germany) according to manufacturer's instructions. The PCR products were obtained using 3 forward primers with annealing temperature of 46°C. The amplified genes appeared at 1191 bp (open reading frame of 205y lipase; ORF), 1095 bp (205y lipase without signal peptide; -SP) and 900 bp (205y lipase without signal peptide and transmembrane domain; -SP/TM) as shown in Figure 4. The idea of removing the signal peptide and transmembrane domain lies on the fact that hydrophobicity nature of both segments is most likely to diminish the lipolytic activity of 205y lipase due to the formation of protein aggregates.

### Sub-cloning of 205y Lipase Genes into pBAD and Transformation into E. coli TOP10

The purified PCR products of 205y lipase; ORF (1191 bp), -SP (1095 bp) and -SP/TM (900 bp) were cloned into pBAD expression vector using pBAD TOPO^{®} TA Kit (Invitrogen, USA). Freshly purified PCR products (4 µL), pBAD TOPO^{®} vector (2 µL) and salt solution (2 µL) were gently mixed and left at room temperature for 5 min.

The reaction mixture (2 µL) was then added to *E. coli* TOP10 competent cells. The mixture was incubated on ice for 5-30 min, heat shock at 42°C for 30 sec without shaking and immediately transferred onto ice. Subsequently, SOC medium (250 µL) was added to the mixture and followed with 1 h incubation at 37°C under shaking condition (200 rpm). Transformed cells were then plated onto pre-incubated LB/tributyrin agar supplemented with 50 µg/mL ampicillin and incubated overnight at 37°C.

Three PCR products, estimated to be 1191 bp (ORF), 1095 bp (-SP) and 900 bp (-SP/TM) in sizes, were successfully sub-cloned into pBAD using pBAD TOPO^{®} TA Expression Kit from Invitrogen, USA. The plasmids, designated as pBAD/ORF, pBAD/-SP and pBAD/- SP/TM were then transformed into *E. coli* TOP10 for lipase expression.

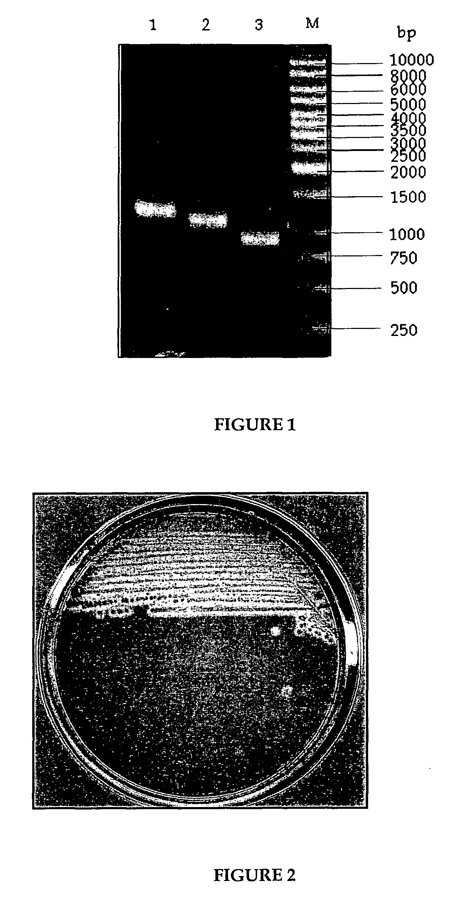

Subsequently, the transformants were screened on LB agar supplemented with tributyrin (5% v/v) and ampicillin (50 µL/mL) for lipolytic activity. After an overnight incubation at 37°C, a considerable number of *E. coli* TOP10 colonies were seen on plates. However, only colonies harbouring pBAD/-SP/TM were surrounded with clearing zones. The presence of clearing zones on LB/tributyrin agar implied a positive lipolytic activity of this transformant (**Figure 2**).

Colonies of *E. coli* TOP10 harboring pBAD/ORF, pBAD/-SP and pBAD -SP/TM were randomly picked for further analysis. Here, plasmids were extracted and the circular forms of them are shown in **Figure 3****.** Different sizes were detected as all plasmids presence in supercoiled form. Analysis of the positive transformants was then carried out using PCR and sequencing approaches.

### Analysis of Positive Transformants

### PCR Technique

The transformants of pBAD/ORF, pBAD/-SP and pBAD/-SP/TM were inoculated into 10 mL LB broth supplemented with ampicillin (50 µg/mL). The broth were grown overnight at 37°C with agitation rate of 200 rpm. The plasmids were then extracted using QIAprep^{®} Miniprep Spin Kit (Qiagen, Germany) according to manufacturer's instructions. The plasmids were electrophoresed on 1% (w/v) agarose gel and visualized under UV radiation after staining with ethidium bromide (1 mg/mL) for 10-15 min.

Subsequently, PCR was carried out using the extracted plasmids as DNA template. Two sets of primers were used for amplification of the transformants (**Table 3**) and the reaction mixtures as well as its condition were as described previously. The PCR products were analyzed by 1% (w/v) agarose gel and the size was estimated based on 1kb DNA Ladder (MBI Fermentas, USA).

**Table 3. Combination of primers for analysis of transformants**

| | Forward | Reverse |
|---|---|---|
| Set 1 | 205y lipase (ORF, -SP and -SP/TM) | 205y lipase |
| Set 2 | 205y lipase (ORF, -SP and -SP/TM) | pBAD |

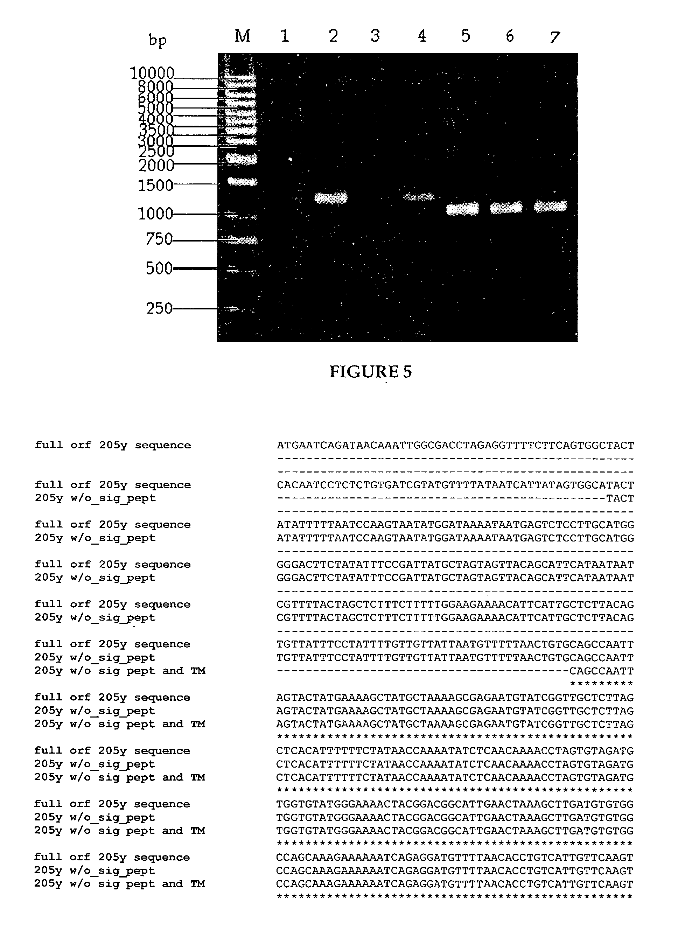

PCR is a preliminary study carried out for determination of positive transformants as it offers highly sensitive yet less time consuming amplification (Miles *et al.,* 1998). The PCR technique confirmed the correct orientation of 205y lipase genes with the C-terminal V5 epitope and 6xHis tag. Two sets of primers were used; 205y lipase forward (ORF, -SP and-SP/TM) combined with 205y lipase reverse (1^{st} set) and 205y lipase forward (ORF, - SP and -SP/TM) combined with pBAD reverse (2^{nd} set). The first combination of primers verified the presence of inserts whereas the second combination confirmed the orientation of constructs. Results are shown in **Figure 4** **and** **5****.** Based on the results obtained, it was proven that almost all 205y lipase genes constructed were in framed with the C-terminal V5 epitope and 6xHis tag of pBAD sequence, except one *E. coli* colony which harbored pBAD/-SP.

### _{Sequencing of 205y Lipase Genes}

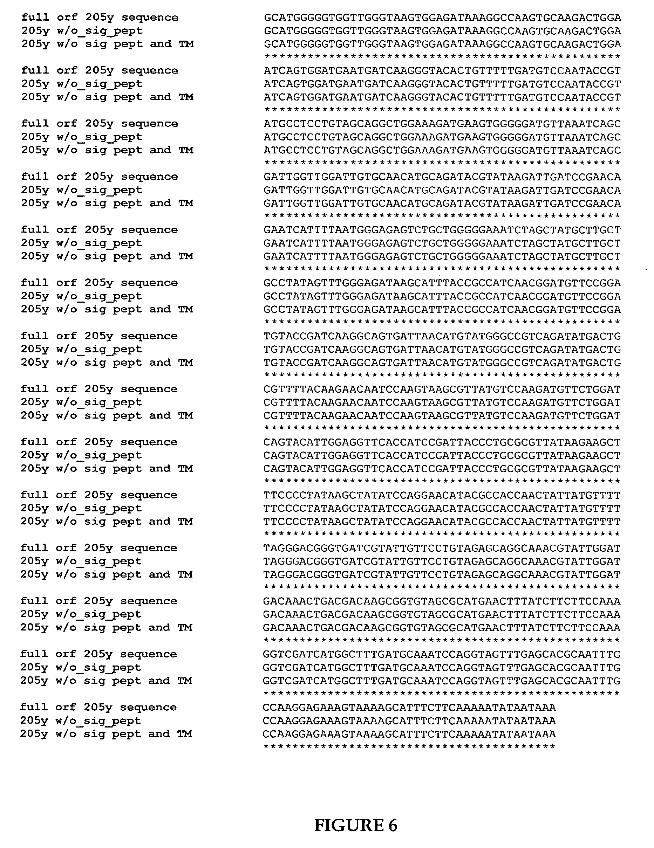

The purified PCR products were sent for automated sequencing (Applied Biosystem, USA) to confirm that 205y lipase genes were successfully cloned with a correct orientation and both, signal peptide as well as transmembrane domain were successfully removed from the open reading frame. Sequence analysis was carried out using the Blast from National Center of Biotechnology (http://www.ncbi.gov) and Expasy Molecular Biology server (http://www.expasy.com). The multiple sequence alignment was then performed using ClustalW program (http://workbench.sdsc.edu/). **Figure 6** reveals the sequencing results of 205y lipase genes. The ORF, -SP and -SP/TM consisted of 1191, 1095 and 900 nucleotides, respectively. As good alignment of the 3 genes was obtained, it therefore reflected the successful alterations of the open reading frame of 205y lipase which were performed beforehand.

### Expression of pBAD/205y Lipase in E. coli TOP10

A single colony of *E. coli* TOP10 harboring pBAD/205y lipase was grown in 50 mL LB broth containing ampicillin (50 µg/mL) to an absorbance of 0.5-0.6 at 600 nm. The culture was then induced with 0.02% L-arabinose and incubation continued for 4 h at 37°C. Consequently, cells were harvested by centrifugation at 10, 000 rpm for 10 min at 4°C, resuspended and incubated in 50 mM phosphate buffer, pH 7.0, followed by sonication with Branson Sonifier 250 to release intracellular proteins. The cell-free extracts were centrifuged at 10, 000 rpm for 10 min to remove cell debris and assayed for lipase activity. Transformants with highest lipase activity was chosen for further analysis. The specific activity of 205y lipase (ORF, -SP and -SP/TM) is shown in **Table 4.** The activities measured for pBAD/ORF and pBAD/-SP were considered low compared to pBAD/- SP/TM.

**Table 4. Lipolytic activity of 205y lipase**

| | ORF | -SP | -SP/TM |
|---|---|---|---|
| Specific activity (U/mg) | 0.72 ± 0.31 | 0.67 ± 0.22 | 9.32 ± 0.24 |

| | | | |
|---|---|---|---|
| *Mean values standard deviations (n=9) *Mean values is significantly different (p<0.05) | | | |

### Optimization on the Expression of 205y Lipase (-SP/TM)

Optimization on the expression of 205y lipase (-SP/TM) was carried out by cultivating the recombinant cultures harboring pBAD vector into 200 mL LB broth containing ampicillin (50 µg/mL) in 1 L blue cap bottle. After 24 h incubation at 37°C, the culture (10 mL) was centrifuged (10, 000 rpm, 10 min, 4°C) and the pellet was resuspended with 10 mL of 50 mM sodium phosphate buffer (pH 7.0) prior sonication. The cell debris was removed by centrifugation at 10, 000 rpm for 10 min at 4°C. The clear supernatant obtained constituted the intracellular crude enzyme solution. E. *coli* TOP10 has been extensively used as hosts for the production of heterologous proteins due to their ability to grow rapidly plus their availability of growing at high density on inexpensive substrates. In fact, its genetics are far well characterized rather than other microorganism (Baneyx, 1999). The expression of 205y lipase (-SP/TM) in *E. coli* was optimized by considering several parameters including inducer's (L-arabinose) concentration, time of induction and induction at A₆₀₀ₙₘ.

### Concentration of Inducer

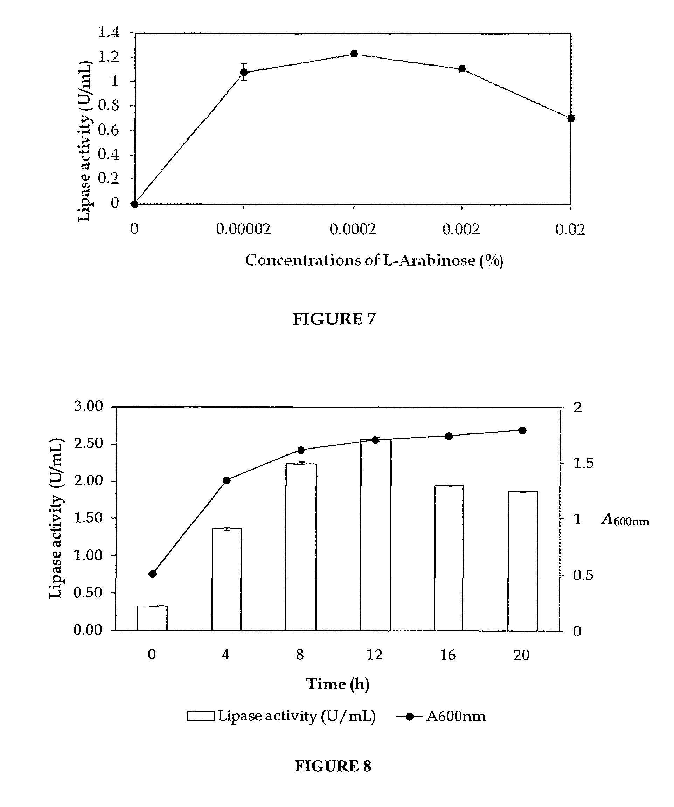

*E. coli* TOP10 harboring recombinant plasmid pBAD/205y lipase (-SP/TM) was induced with different concentration of L-arabinose (0.02%, 0.002%, 0.0002% and 0.00002%) at *A*₆₀₀ₙₘ ∼0.5 for 24 h cultivation time (Invitrogen, USA). Subsequently, the intracellular enzyme was subjected to lipase assay. 205y lipase (-SP/TM), the concentration of L-arabinose for expression was investigated by varying the concentrations from 0.00002% to 0.02%. Results obtained suggested that 0.0002% of L-arabinose was sufficient to enhance the expression of 205y lipase (-SP/TM) whereby higher and lower concentrations depressed its expression (**Figure 7**). Principally, upon entering the cell, L-arabinose would be converted sequentially to simpler sugars, and further catabolized through the pentose phosphate shunt (Sá-Nogueira and Mota, 1997). Therefore, in order to achieve an excellent catabolic system of pBAD in *E. coli,* the amount of L-arabinose used to induce the promoter should be optimized

### Induction Time

*E. coli* TOP10 harboring pBAD/205y lipase (-SP/TM) was induced with 0.02% L-arabinose and cultivated up to 20 h. The expression of 205y lipase (-SP/TM) was deliberately monitored every 4 h intervals, ranging from 0-20 h. Lipase assay was then carried out on the intracellular enzyme. The optimized induction time was applied for the subsequent studies. *E. coli* TOP10 harboring pBAD/-SP/TM was induced with 0.0002% L-arabinose and concurrently incubated at 37°C up to 20 h.

The culture reached maximum level of expression at 12 h induction with intracellular lipase activity of 1.70 U/mL (**Figure 8**). On the whole, the secretion of protein went in parallel with the bacterial growth curve (*A*₆₀₀ₙₘ). However, at the end of the induction, the expression declined as the bacterial growth went stagnant (quantity of alive equalled to the dead bacteria).

### Induction at A₆₀₀ₙₘ

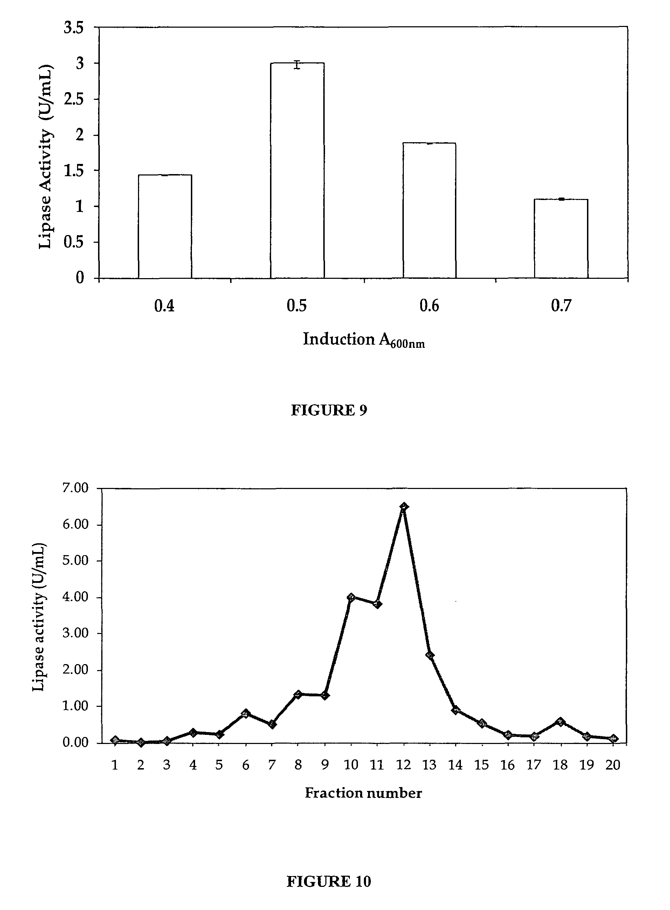

*E. coli* TOP10 harboring pBAD/205y lipase (-SP/TM) was induced with 0.0002% L-arabinose at different *A*₆₀₀ₙₘ ranging from 0.4-0.7. The culture was grown for 12 h at 37°C and after centrifugation (10, 000 rpm, 10 min, 4°C), the intracellular enzyme was subjected to lipase assay. Induction at *A*₆₀₀ₙₘ played an important role in expression of protein by *E. coli* TOP10 harboring pBAD/-SP/TM. Based on the results obtained, induction at 0.5 of *A*₆₀₀ₙₘ increased 205y lipase (-SP/TM) activity. In contrast, 1.88 U/mL of lipase activity was detected at 0.6 of *A*₆₀₀ₙₘ, whereby 1.10 U/mL was detected at 0.70 as shown in **Figure 9****.** *E. coli* that harbors different plasmids will have different induction at *A*₆₀₀ₙₘ. For instance, for *E. coli* harboring pGEX/T1S, the highest lipase activity was achieved after induction at 0.75 of *A*₆₀₀ₙₘ. In contrast, for *E. coli* harboring pJL3/T1S, the secretion of lipase reached maximal level after induction at 1.25 of *A*_{600 nm} (Leow, 2005). Owing to the variations, *E. coli* remains as a valuable tool for the production of recombinant proteins, to date.

### Quantitative Determination of Lipase Activity

### Lipase Assay

Lipolytic activity was measured by the method of Kwon and Rhee (1986) with slight modifications. The reaction was started with the preparation of olive oil-phosphate buffer (1:1 v/v) emulsion. Crude cell lysate (0.1 mL) was then shaken with 2.5 mL olive oil emulsion and 20 µL of 20 mM CaCl₂ in water bath shaker (37°C) at agitation rate of 200 rpm for 30 min. The enzyme reaction was terminated by the addition of 1.0 mL 6 M HCl while 5.0 mL isooctane was added to the reaction mixture to extract the free fatty acids produced by olive oil hydrolysis, followed by mixing using a vortex mixer for 30 sec.

The upper layer (4.0 mL) was transferred to test tube and 1.0 mL of copper acetate pyridine was added to yield a green colour which corresponds to the production of free fatty acids. The absorbance was read at 715 nm after 1 h incubation at room temperature. The amount of free fatty acids was determined based on the standard curve of free fatty acids. One unit (U) of lipase activity is equivalent to the rate of fatty acids formation (µmole) per min (standard assay condition).

### Standard Curve of Oleic Acid

A standard curve of oleic acid was prepared using oleic acid standard solutions ranging from 0.0-31.5 µmole (Appendix A). Different concentrations of oleic acid were prepared using a stock solution of oleic acid (0.1 mL in 9.9 mL of isooctane) and details of the mixture are shown below:

| Tube | Stock of oleic acid (mL) | Amount of oleic acid (µmole) | Isooctane (mL) | Copper reagent (mL) |
|---|---|---|---|---|
| 0 | 0.0 | 0.0 | 5.0 | 1.0 |
| 1 | 0.2 | 6.3 | 4.8 | 1.0 |
| 2 | 0.4 | 12.6 | 4.6 | 1.0 |
| 3 | 0.6 | 18.9 | 4.4 | 1.0 |
| 4 | 0.8 | 25.2 | 4.2 | 1.0 |
| 5 | 1.0 | 31.5 | 4.0 | 1.0 |

### Purification of Recombinant 205y Lipase (-SP/TM)

### Isoelectric Focusing Using Rotofor^{®} System

Two hundred mL of recombinant *E. coli* harbouring 205y lipase was harvested by centrifugation (10, 000 rpm, 10 min, 4°C) and subsequently dissolved with 10 mM tris-HCl buffer, pH 7.2 (50 mL) prior to sonication. The crude cell lysate was then mixed with Bio-Lyte ampholyte (40% w/v) and subsequently loaded into the filling ports of the focusing chamber using 50 mL syringe with a 1-1/2 inch 19-gauge needle. Rotofor^{®} was run at constant power of 15.0 W whilst the temperature of the focusing chamber was maintained at 4°C. Fractions containing the protein of interest were collected after 2-3 h rotation.

### Determination of Protein Content

The concentration of protein was spectrophotometrically determined at *A*₅₉₅ₙₘ as described by Bradford (1976) with bovine serum albumin (BSA) as standard. The standard solution of BSA concentrations between 10 µg to 6 µg was used in standard curve preparation. One hundred µL of protein solution was transferred into test tube and added with 50 mM phosphate buffer pH 7.0 to make a total volume of 2.5 mL. Five mL of Bradford working buffer [425 mL distilled water, 15 mL of 95% (v/v) ethanol, 30 mL 88% (v/v) phosphoric acid, and 30 mL Bradford stock solution (100 mL 95% ethanol, 200 mL 88% phosphoric acid, 350 mg Serva Blue G)] was then added to the protein solution, followed by vigorous vortex for 5 sec. Absorbance was read after 10 min using spectrophotometer.

### Purity and Molecular Weight Estimation of 205y Lipase (-SP/TM)

### a) Polyacrylamide Gel Electrophoresis

The purity and relative molecular mass of the eluted proteins that gave highest lipase activity were estimated using 12% gradient gel of SDS-PAGE under the conditions developed by Laemmli (1970) (**Table 5**). Gels were subsequently stained with Coomassie brilliant blue R-250. A broad range of protein standard was used as molecular mass marker.

**Table 5: Composition for SDS-PAGE [12% (w/v) gel separation]**

| Composition | Separating gel [12% (w/v)] | Stacking gel [6% (w/v)] |
|---|---|---|
| Distilled water | 4.4 mL | 5.9 mL |
| 40% Bis-acrylamide | 3.0 mL | 1.5 mL |
| 1.5 M Tris-HCl buffer (pH 8.8) | 2.5 mL | - |
| 0.5 M Tris-HCl buffer (pH 6.8) | - | 2.5 mL |
| 10% (v/v) SDS | 0.1 mL | 0.1 mL |
| 10% (w/v) APS (fresh preparation) | 50 µL | 50 µL |
| TEMED | 10 µL | 15 µL |

### b) Activity Staining

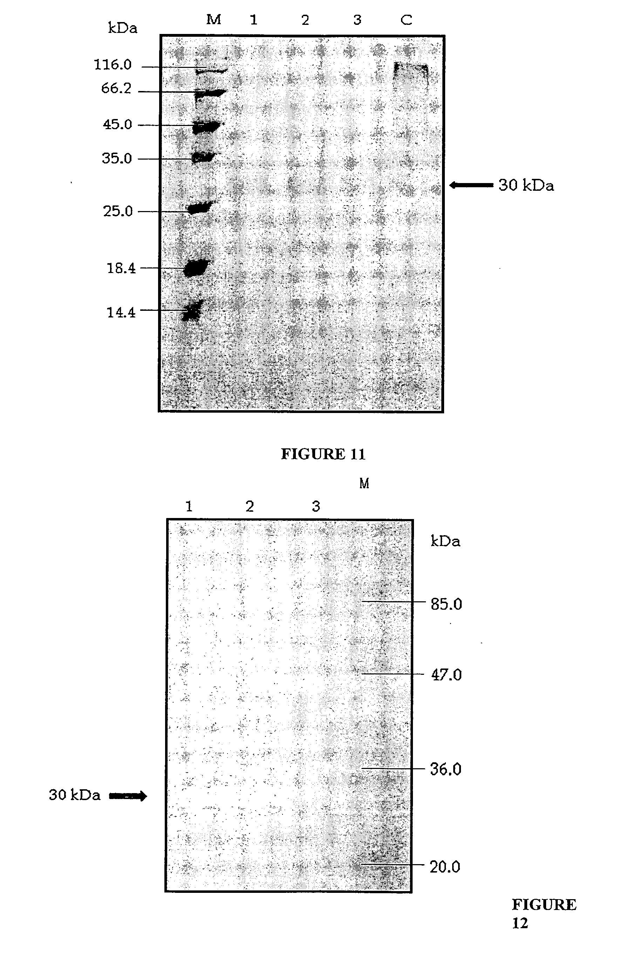

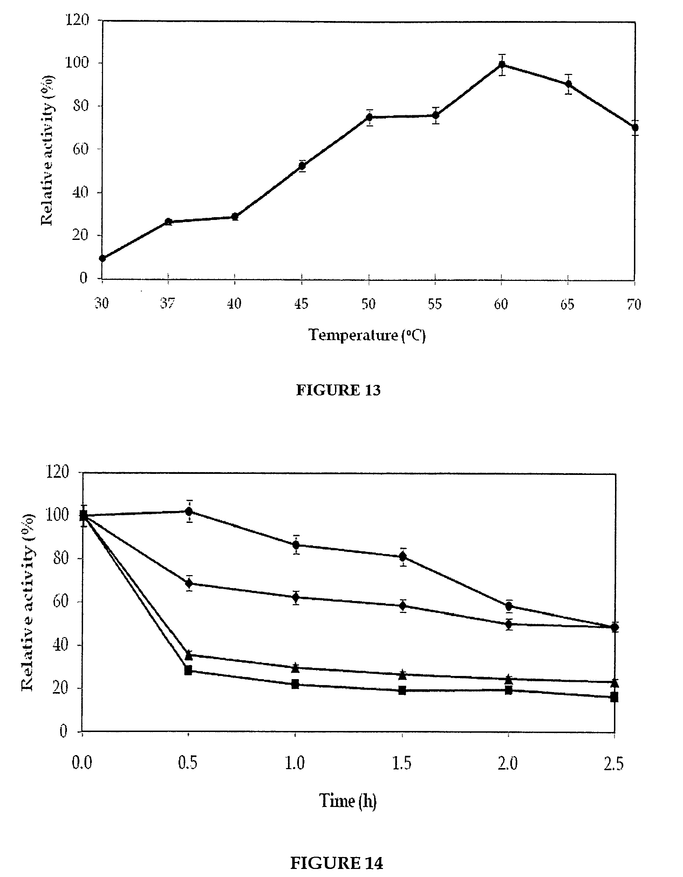

The purified native proteins were loaded into SDS-PAGE 12% (w/v) gel. After electrophoresis, gel was immersed in 20% (v/v) isoproponol to remove the SDS and subsequently washed with distilled water (2-3 times) before transferred onto LB/tributyrin agar supplemented with ampicillin (50 µg/mL). The plate was incubated at 37°C for 4-5 h. The presence of clearing zones represented positive lipolytic activity. The prestained protein molecular weight marker (MBI Fermentas, USA) was used to estimate the molecular mass of the protein. The crude recombinant 205y lipase (-SP/TM) was purified to homogeneity using isoelectric focusing technique, facilitated by Rotofor^{®} system. The Rotofor^{®} cell is a unique preparative protein purification device that separates proteins in free solution by liquid-phase isoelectric focusing (IEF). This purification procedure was carried out on the basis of the amphoteric nature of proteins; PI which allow the separation by IEF. The protein sample was mixed with Bio-Lyte ampholytes as it allowed the Rotofor^{®} system to generate linear pH gradients to fractionate complex mixtures of protein (Ayala *et al.,* 1998). The purified protein was then harvested in free solution within specific pH intervals. The mixture of crude cell lysates of 205y lipase (-SP/TM) and Bio-Lyte ampholytes (pH 3-10) was successfully purified after 3 h rotation. At this point, 20 fractions were collected with each of the fractions represented different PIs. All fractions were then subjected for lipase assay and the purification profile is shown in **Figure 10****.** Fractions with the highest lipolytic activity were further analyzed using SDS-PAGE and activity staining (**Figure 12** **and** **13**). The purification results are summarized in **Table 6.**

**Table 6: Summary of the purification of 205y lipase (-SP/TM)**

| Purification step | Total activity (U) | Total protein (mg) | Specific activity (U/mg) | Recovery (%) | Purification fold |
|---|---|---|---|---|---|
| Crude extract | 427.5 | 30.0 | 14.25 | 100 | 1 |
| Rotofor® system | 227.5 | 14.0 | 16.25 | 53 | 1.1 |

The purification of 205y lipase (-SP/TM) was achieved using pH range of 3-10 with 53% recovery and purification fold of 1.1. The most challenging part upon using this purification technique was to maintain the solubility of protein throughout the purification procedure. Protein at their P*I*s has no net charge, thus little charge repulsion existed between focused molecules.

### Characterization of Purified 205y Lipase (-SP/TM)

### a) Effect of Temperature on Lipase Activity

The effect of temperature on 205y lipase (-SP/TM) activity was investigated using a temperature range of 30-70°C. Purified enzyme (100 µL) was incubated with 2.5 mL substrate [olive oil: phosphate buffer pH 7.0, 1:1, (v/v)] and 20 µL of 20 mM CaCl₂ in water bath shaker at an agitation rate of 200 rpm for 30 min. Lipase activity was measured colorimetrically. Relative activity was expressed as a fraction to the highest activity obtained. The colorimetric measurement was carried out for 30 min to investigate the effect of temperature on the purified 205y lipase (-SP/TM) activity. Various temperatures ranging from 30°C to 70°C were tested. 205y lipase (-SP/TM) was most active between 55°C to 65°C as shown in **Figure 13**. The optimum temperature of this modified lipase was higher than the open reading frame (55°C) as revealed by Sulong *et al.*, 2006. Though 205y lipase was classified under mesophilic group of enzyme, it surprisingly showed highest activity in thermostable range (45-80°C).

Other organic solvent tolerant lipases reported thus far, on the other hand, showed lower optimum temperature compared to 205y lipase (-SP/TM). The organic solvent tolerant lipases from *Psedumonas aeruginosa* LST-03 (Ogino *et al.,* 2000) and *Pseudomonas* sp. S5 (Rahman *et al.,* 2005), for instance, showed optimum temperature at 37°C and 45°C, respectively. However, other lipases, without organic solvent tolerant characteristic have been reported to have as high as 55 to 60°C of optimum temperature as revealed on *Bacillus licheniformis* lipase (Nthangeni *et al.,* 2001) and *Pseudomonas cepacia* lipase by Bornscheuer *et al.* (1994). The temperature profile of 205y lipase (-SP/TM) obtained from this study can be explained on the basis of enzyme denaturing theory. As temperature increased, molecules and ions were kinetically energetic. The high collision frequency between both, molecules and ions therefore increased the reaction rate. However, at optimum temperature or above (60°C), the tertiary structure of this enzyme was destroyed. This was due to the increase in kinetic energy and vigorous vibration which caused the hydrogen bond to break and denatured. As a result, the substrate was no longer able to fit into the active site, hence lowering the activity of 205y lipase (-SP/TM).

### b) Effect of Temperature on Lipase Stability

The effect of temperature on 205y lipase (-SP/TM) stability was determined by preincubating the reaction mixture (as mentioned above) at various temperatures ranging from 37-60°C for 30 min intervals prior to lipase assay. The residual activity was measured at 60°C (optimum temperature) under shaking condition (200 rpm) for 30 min. The treatments of 205y lipase (-SP/TM) with various effectors (metal ions, surfactants and inhibitors) in the subsequent sections were performed at temperature where the enzyme is stable at least 1 h. The optimum temperature was used to perform the lipase assay. The thermostability study of 205y lipase (-SP/TM) was carried out from 37°C to 60°C. This enzyme retained its original activity more than 80% for 1 h 30 min treatment at 37°C as illustrated in **Figure 14****.** At 50°C, 205y lipase (-SP/TM) was able to maintain its activity for 2 h. However, it was found that at higher temperature (55°C and 60°C), this enzyme was not stable at all resulting in the drop of activity as low as 36 and 28%, respectively even after 30 min treatment.

### c) Effect of pH on Lipase Activity

The effect of pH on 205y lipase (-SP/TM) was measured colorimetrically at various pH (pH 4.0-12.0) under agitation rate of 200 rpm for 30 min. The substrate was prepared using 50 mM buffer of different pH values. The following buffers were used in this study: acetate buffer (pH 4.0-6.0), potassium phosphate buffer (pH 6.0-8.0), tris-HCl buffer (pH 8.0-9.0), glycine-NaOH buffer (pH 9.0-11.0) and Na₂HPO₄/NaOH buffer (pH 11.0-12.0).

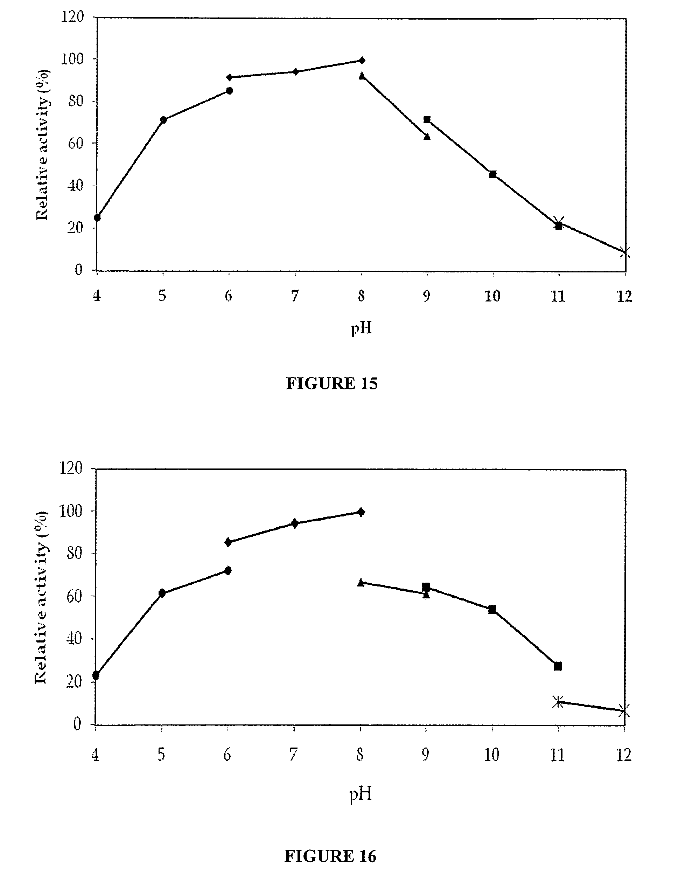

The effect of pH on 205y lipase (-SP/TM) activity was investigated at different pH ranging from pH 4.0 to pH 12.0. It was found that this modified enzyme preferred neutral pH, as reported on the open reading frame (ORF) (Sulong e*t al.*, 2006). More than 50% activity was detected from pH 5.0 to pH 9.0, with the optimal at pH 8.0 in 50 mM potassium phosphate buffer. As pH increased, 205y lipase (-SP/TM) showed instantaneous loss of activity (**Figure 15**).

### d) Effect of pH on Lipase Stability

The pH stability of 205y lipase (-SP/TM) was investigated by preincubating the enzyme with buffers of various pH (1:1, v/v) for 30 min at 200 rpm agitation rate. Residual activity was measured colorimetrically after the pH treatment. The pH stability test showed that 205y lipase (-SP/TM) was relatively stable (>50% activity) at pH ranging from 5.0 to 10.0 upon treatment at 37°C for 30 min in various buffers. Treatment of the enzyme at pH below 5.0 and above 10.0 has resulted in the loss of activity (**Figure 16**). The enzyme activity was not recovered at that pH ranges as the enzyme was not stable due to the alteration of ionic bonds, concurrently destabilizing its 3D structure.

### e) Effect of Organic Solvents on Lipase Activity

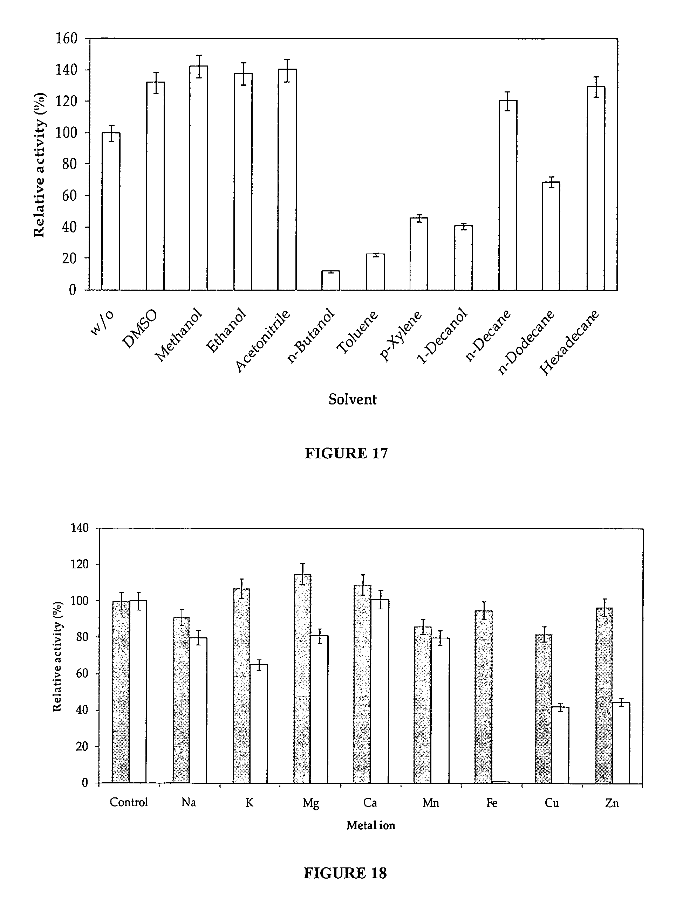

The activity of 205 lipase (-SP/TM) towards the presence of organic solvents was tested by preincubating a mixture of purified enzyme with organic solvents (3:1, v/v) for 30 min at agitation rate of 200 rpm. Residual activity was measured at optimum temperature and expressed as percentage of activity without organic solvent. The effect of organic solvents on purified 205y lipase (-SP/TM) activity was determined using several organic solvents with different log P value ranging from -1.2 to 8.8. Mixture of enzyme and organic solvent (3:1 v/v) was incubated at 37°C for 30 min and the residual activity was measured using spectrophotometric method. As illustrated in **Figure 17**, 205y lipase (-SP/TM) retained its activity in the presence of both, water miscible (low log P) as well as water immiscible (high log P) solvents. 205y lipase (-SP/TM) was stable in the presence of DMSO (log P - 1.2), methanol (log *P* -0.76), ethanol (log *P* -0.24), acetonitrile (log *P* -0.15), *n*-decane (log *P* 5.6) and hexadecane (log *P* 8.8) with more than 30% enhancement in activity. In contrast, the presence of *n*-butanol (log *P* 0.8), toluene (log *P* 2.5), *p*-xylene (log *P* 3.1), 1-decanol (log *P* 4.0) and *n*-dodecane (log *P* 6.6) inhibited the enzyme with more than 50% lost in activity. Basically, the effect of organic solvents on lipases has no specific trend as different lipases showed different degree of tolerance towards organic solvents. The tendency of solvents in destabilizing enzymes does not depend solely on their hydrophobicity, since other physicochemical characteristics of solvents such as the solvating ability and molecular geometry are of important.

### f) Effect of Metal Ions on Lipase Activity

The effect of metal ions on lipase activity was investigated by treating 205y lipase (-SP/TM) with 1 mM and 10 mM of various metal chlorides ( Na⁺, K⁺, Mg²⁺, Ca²⁺, Fe²⁺, Mn²⁺, Zn²⁺ and Cu²⁺) in 50 mM potassium phosphate buffer pH 8.0 at 37°C for 30 min. The mixture was subjected to lipase assay after treatment and expressed as a percentage of activity without the metal chloride. As shown in **Figure 18****,** the activity was promoted in the presence of K⁺, Mg²⁺ and Ca²⁺ (1 mM) whereas Na⁺, Mn²⁺, Fe²⁺, Cu²⁺ and Zn²⁺ (1 mM) slightly decreased the activity (>50% activity retained). Moreover, higher concentration of ions (10 mM) also decreased the lipase activity.

Since the activity of 205y lipase (-SP/TM) was enhanced in the presence of K⁺, Mg²⁺ and Ca²⁺, therefore this lipase can be classified as metal-activated enzyme. Having said so, ions in metal-activated enzyme play important role in structural rather than catalytic reactions. Here, the ions bind to the enzyme and change its conformation to give a greater stability. Transition metal ions, however, change the conformation to less stable form due to ion toxicity (Rahman *et al.*, 2005). This somewhat explained the inhibition caused by Mn²⁺, Fe²⁺, Cu²⁺ and Zn²⁺ to 205y lipase (-SP/TM) activity.

### g) Effect of Surfactants on Lipase Activity

The activity of 205y lipase (-SP/TM) towards surfactants was determined by preincubating the enzyme with various surfactants at a concentration of 1 mM at 37°C for 30 min. The surfactants used were Tween 20, Tween 40, Tween 60, Tween 80, Triton X-100, sodium dodecyl sulphate (SDS) and sodium lauryl sulphate (SLS). Residual activity was measured colorimetrically and expressed as percentage of activity without surfactant. In general, all surfactants inhibited the enzyme with less than 50% lipase activity detected, except for SLS with residual activity of 52.5% (**Table 7**).

**Table 7. Effect of surfactants on 205y lipase (-SP/TM) activity**

| Surfactant | Relative activity (100%)* |
|---|---|
| Control | 100.0 ± 0.5 |
| Tween 20 | 31.3 ± 1.1 |
| Tween 40 | 27.9 ± 1.7 |
| Tween 60 | 34.0 ± 3.5 |
| Tween 80 | 29.4 ± 2.4 |
| Triton X-100 | 47.3 ± 4.4 |
| SDS | 28.9 ± 2.8 |
| SLS | 52.5 ± 1.9 |

| | |
|---|---|
| Note: The 205y lipase was incubated with 0.1% surfactant at 37°C for 30 min prior to lipase assay. * Relative activities are mean values ± standard deviations of three determinations (n=3). | |

### h) Effect of Inhibitors on Lipase Activity

The effect of inhibitors were tested on 205y lipase (-SP/TM) activity by preincubating the enzyme with dithiothreitol (DTT), β-mercaptoethanol, phenylmethylsulfonyl fluoride (PMSF), ethylenediaminetetraacetic acid (EDTA) and pepstatin A at different concentrations (1 and 5 mM) except for pepstatin A (1 mM). The residual activity was measured at optimum temperature and expressed as percentage of activity without inhibitor. The reducing agents, β-mercaptoethanol and DTT showed slight inhibition to the enzyme activity with 2.8 and 16.6% inhibition, respectively at 1 mM concentrations. This results indicated that 205y lipase (-SP/TM) lacks the disulphide bond, which of important in the structural based. The metal chelating agent, EDTA also showed a slight inhibition to 205y lipase (-SP/TM) with remaining activity of 78.2% upon treatment. This proved that this lipase was not a metalloenzyme. Table 8 provides the effect of inhibitors on 205y lipase (-SP/TM) activity.

**Table 8**

| | Relative activity (100%)* | |
|---|---|---|
| | 1 mM | 10 mM |
| Control | 100 ± 0.3 | 100 ± 0.3 |
| β-mercaptoethanol | 97.2 ± 2.7 | 76.3 ± 1.2 |
| DTT | 83.4 ± 2.0 | 45.5 ± 2.5 |
| EDTA | 78.2 ± 1.8 | 40.1 ± 4.2 |
| PMSF | 74.0 ± 3.3 | 70.3 ± 2.8 |
| Pepstatin A | 86.9 ± 1.5 | - |

| | | |
|---|---|---|
| Note: The 205y lipase was incubated with 1 mM and 10 mM inhibitor (except for pepstatin A) at 37°C for 30 min prior to lipase assay. * Relative activities are mean values ± standard deviations of three determinations (n=3). | | |

### i) Substrate Specificity Towards Triacylglycerols and Natural Oils

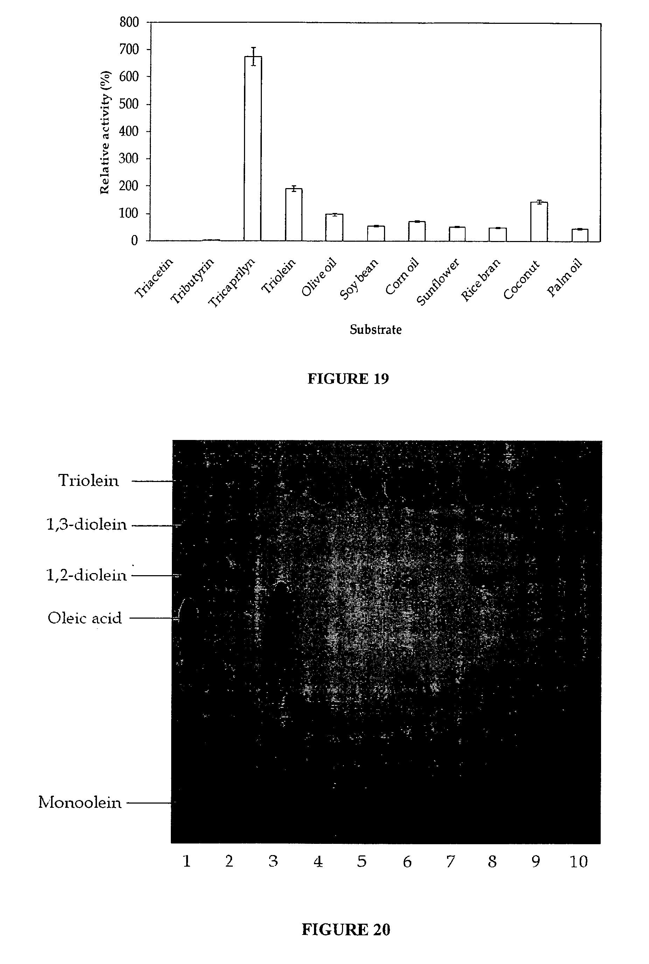

Lipolytic activity of the purified lipase towards triacylglycerols and natural oils was determined. Various triacylglycerols (triacetin, tributyrin, tricaprilyn, triolein) and natural oils (olive oil, soy bean oil, sun flower oil, rice bran oil, coconut oil and palm oil) were used as substrates in the assay reaction. Relative activity was measured as a fraction to the highest activity obtained. **Figure 19** shows 205y lipase (-SP/TM) demonstrated a preference against both tested substrates. However, highest relative activity was detected on tricaprilyn (C8), 676% and triolein (C18), 192%.

### i) Positional Specificity of 205y Lipase (-SP/TM)

The regioselectivity of 205y lipase (-SP/TM) was determined as described by Ogino *et al.*, (2000) with slight modifications. Mixture of purified lipase, 100 mM triolein and 50 mM potassium phosphate buffer pH 8.0 was incubated at 37°C for 30 min. The reaction products were then extracted with 5 mL of n-hexane and analyzed using thin layer chromatography (TLC). A silica gel plate 60 (Merck, Germany) was developed in a mixture of chloroform, acetone and acetic acid (96:4: 1). The spots of glycerides and fatty acids were visualized by exposure to iodine vapour. The commercial enzymes, Lipozyme and Novozyme were included in this experiment as controls, where the positional specificity has been verified.

Breakdown of the pure triolein to 1,2-diolein by 205y lipase (-SP/TM) was observed at the end of the experiment. This product was similar to Lipozyme (1,3-regiospecific) as shown in **Figure 20****.** Therefore, 205y lipase (-SP/TM) can be classified as 1,3-regiospecific lipase.

Therefore the present invention further comprises:
1. Novel organic solvent tolerant lipase gene obtained from a biologically pure culture of *Bacillus sphaericus,* wherein the lipase gene includes 3 lipase gene(s) of SEQ ID NO 1, SEQ ID NO 2 and SEQ ID NO 3
2. The novel organic solvent tolerant lipase gene according to claim 1, wherein the lipase gene is 205y lipase.
3. A method of obtaining organic solvent 205y lipase genes, the method provides the means of using primers to amplify the genes by polymerase chain reaction (PCR) technique.
4. The method according to claim 3, wherein the primers to amplify the genes, wherein the primers includes
   d) 5'-ATG AAT CAG ATA ACA AAT TGG-3'(SEQ ID NO4)
   e) 5'-TAC TAT ATT TTT AAT CCA AGT AAT-3'(SEQ ID NO5
   f) 5'-CAG CCA ATT AGT ACT ATG AAA AGC-3'(SEQ ID NO6)
5. The method according to claim 4, wherein the primers with SEQ ID NO4 having to amplify the 205y lipase gene with an open reading frame.
6. The method according to claim 5, wherein the primers with SEQ ID NO4 having to amplification size of 1191 base pair.
7. The method according to claim 4, wherein the primers with SEQ ID NO5 having to amplify the 205y lipase gene without a signal peptide.
8. The method according to claim 7, wherein the primers with SEQ ID NO5 having to amplification size of 1095 base pair.
9. The method according to claim 4, wherein the primers with SEQ ID NO6 having to amplify the 205y lipase gene without a signal peptide and transmembrane domain.
10. The method according to claim 9, wherein the primers with SEQ ID NO6 having to amplification size of 900 base pair.
11. A purified 205y lipase without signal peptide and transmembrane domain is obtained from the preceding method claim 3 -10, wherein purified 205y lipase without signal peptide and transmembrane domain including the steps of:
   a) harvesting recombinant *E. coli* by centrifugation at 10 rpm, for 10 min at 4°C;
   b) obtaining pellet from step (a) and dissolving the pellet in tris-HCL buffer (10mM) at pH between 7-7.3;
   c) performing sonication of the pellet and obtaining cell lysate;
   d) mixing the cell lysate with Bio-Lyte ampholyte (40% w/v);
   e) obtaining a mixture of cell lysate with ampholyte and loading the mixture of cell lysate with ampholyte into a protein separation chamber;
   f) obtaining purified fractions of protein.
12. A method of characterizing purified 205y lipase without a signal peptide and transmembrane domain, wherein the method includes the steps of:
   a) analysing temperature on lipase activity and stability;
   b) analysing pH on lipase activity and stability using buffers, wherein the buffers comprises acetate buffer (pH 4.0-6.0), potassium phosphate buffer (pH 6.0-8.0), tris-HCl buffer (pH 8.0-9.0), glycine-NaOH buffer (pH 9.0-11.0) and Na₂HPO₄/NaOH buffer;
   c) analysing organic solvents on lipase activity, wherein the organic solvents comprises DMSO, methanol, ethanol, acetonitrile, *n*-decane, hexadecane, *n-*butanol, toluene, *p*-xylene, 1-decanol and *n*-dodecane ;
   d) analysing metal ion on lipase activity, wherein the metal ions includes K⁺, Mg²⁺ Ca²⁺, Na⁺ M²⁺, Fe²⁺, C²⁺ and Zn²⁺;
   e) analysing surfactants on lipase activity; wherein the surfactants comprises Tween 20, Tween 40 Tween 60 Tween 80, Triton X-100, SDS and SLS;
   f) analysing inhibitors on lipase activity; wherein the inhibitors comprises dithiothreitol (DTT), β-mercaptoethanol, phenylmethylsulfonyl fluoride (PMSF), ethylenediaminetetraacetic acid (EDTA) and pepstatin A;
   g) analysing substrate specificity of the 205y lipase, wherein the substrate comprises triacylglycerols (triacetin, tributyrin, tricaprilyn, triolein) and natural oils (olive oil, soy bean oil, sun flower oil, rice bran oil, coconut oil and palm oil);
   h) determining positional specificity of the 205y lipase.
13. The method of characterizing purified 205y lipase according to claim 12 (a), wherein the method further comprising the steps of:
   a) preparing a reaction mixture by mixing 100 µL of purified enzyme with 2.5 mL substrate [olive oil: phosphate buffer pH 7.0, 1:1, (v/v)] and 20 µL CaCl_{2;}
   b) heating the reaction mixture from step (a) at temperature between 30 °C and 70°C for 30 mins;
   c) agitating the reaction mixture from step (b) at 200 revolution per min(rpm);
   d) measuring lipase activity;
   e) allowing the reaction mixture from step (b) to pre-incubate at temperature between 37 °C and 60°C for 30 mins;
   f) agitating the reaction mixture from step (e) at 200 (rpm) at 60°C; and
   g) measuring lipase activity
   h) The method of characterizing purified 205y lipase according to claim 12 (b), wherein the method further comprising the steps of;
   i) preparing a reaction mixture by mixing 100 µL of purified enzyme with 2.5 mL substrate [olive oil: the buffers between pH 4 and 12, 1:1, (v/v)] and 20 µL CaCl_{2;}
   j) heating the reaction mixture from step (a) at temperature between 30 °C and 70°C for at least 30 mins;
   k) agitating the reaction mixture from step (b) at 200 revolution per min(rpm);
   1) measuring lipase activity;
   m) allowing the reaction mixture from step (b) to pre-incubate with buffers at temperature at 37 °C between pH 4and 12 for at least 30 mins;
   n) agitating the reaction mixture from step (e) at 200 (rpm) at 60°C; and
   o) measuring lipase activity
14. The method of characterizing purified 205y lipase according to claim 12 (c), wherein the method further comprising the steps of:
   a) mixing purified enzyme with individual organic solvents (3:1 v/v) for at least 30 mins for pre-incubation;
   b) agitating the reaction mixture from step (a) at 200 (rpm); and
   c) measuring lipase activity
15. The method of characterizing purified 205y lipase according to claim 12 (d), wherein the method further comprising the steps of:
   a) mixing purified enzyme with individual metal ions according to claim 12(d), wherein the concentration of the metal ions between (1 and 10mM) and the concentration of purified lipase is between 1mM;
   b) obtaining a reaction mixture;
   c) adding potassion phosphate buffer of pH 8 into the reaction mixture from step (b) and pre-incubating the reaction mixture for at least 30 mins at 37 °C;
   d) agitating the reaction mixture from step (c) at 200 (rpm); and
   e) measuring lipase activity
16. The method of characterizing purified 205y lipase according to claim 12 (e), wherein the method further comprising the steps of:
   a) mixing purified enzyme with individual surfactants according to claim 12(e), wherein the concentration of the surfactants between 1mM and the concentration of purified lipase is between 1mM;
   b) obtaining a reaction mixture;
   c) pre-incubating the reaction mixture from step (a) for at least 30 mins at 37 °C;
   d) agitating the reaction mixture from step (c) at 200 (rpm); and
   e) measuring lipase activity
17. The method of characterizing purified 205y lipase according to claim 12 (f), wherein the method further comprising the steps of:
   a) mixing purified enzyme with individual surfactants according to claim 12(f), wherein the concentration of the surfactants (between 1 and 5mM) and the concentration of purified lipase is between 1mM;
   b) obtaining a reaction mixture;
   c) pre-incubating the reaction mixture from step (a) for at least 30 mins at 37 °C;
   d) agitating the reaction mixture from step (c) at 200 (rpm); and
   e) measuring lipase activity.
18. The method of characterizing purified 205y lipase according to claim 12(g), wherein the method further comprising the steps of:
   a) mixing purified enzyme with individual surfactants according to claim 12(g), wherein the concentration of the surfactants between 2.5 mL and the concentration of purified lipase is between 100µL pH 8.0, 1:1, (v/v)] and 20 µL CaCl₂;
   b) obtaining a reaction mixture;
   c) pre-incubating the reaction mixture from step (a) for at least 30 mins at 37 °C;
   d) agitating the reaction mixture from step (c) at 200 (rpm); and
   e) measuring lipase activity
19. The method of characterizing purified 205y lipase according to claim 12 (h), wherein the method further comprising the steps of:
   a) incubating 100µL of purified lipase with 100mM triolein and 50mM of potassium phosphate buffer between pH 8.for at least 30 mins at 37 °C;
   b) obtaining a reaction product of triolein from step (a) by means of extracting the reaction product with 5mL of n-hexane;
   c) obtaining an extract from step step (b) and analysing the extract by using a chromatography;
   d) mixing the extract with a mixture of chloroform, acetone and acetic acid and obtaining a silica gel and
   e) obtaining spots of glycerides and fatty acids by exposing the silica gel from step (d) to iodine vapour.
20. The purified 205y lipase without a signal peptide and transmembrane domain having the means of providing an activity between 55 °C and 65 °C.
21. The purified 205y lipase without a signal peptide and transmembrane domain having the means of providing stability at pH 5 and 10.
22. The purified 205y lipase without a signal peptide and transmembrane domain having the means of providing stability with organic solvents such as DMSO, methanol, ethanol, acetonitrile, n-decane, hexadecane.
23. The purified 205y lipase without a signal peptide and transmembrane domain having the means of providing an activity in the presence of K⁺, Mg²⁺ and Ca²⁺.
24. The purified 205y lipase without a signal peptide and transmembrane domain having the means of providing an activity in the presence of Tween 20, Tween 40 Tween 60 Tween 80, Triton X-100 and SDS.
25. The purified 205y lipase without a signal peptide and transmembrane domain having the means of providing an activity in the presence hydrolyzing triglycerides and natural oils at position 1 and 3.

## Claims

1. Novel organic solvent tolerant lipase gene obtained from a biologically pure culture of *Bacillus sphaericus,* wherein the lipase gene comprises a sequence selected from the group consisting of the sequences shown in SEQ ID NO 1, SEQ ID NO 2 and SEQ ID NO 3

2. The novel organic solvent tolerant lipase gene according to claim 1, wherein the lipase gene is 205y lipase.

3. A method of obtaining organic solvent 205y lipase genes, the method provides the means of using primers to amplify the genes by polymerase chain reaction (PCR) technique.

4. The method according to claim 3, wherein the primers to amplify the genes, wherein the primers includes
a) 5'-ATG AAT CAG ATA ACA AAT TGG-3'(SEQ ID NO4)
b) 5'-TAC TAT ATT TTT AAT CCA AGT AAT-3'(SEQ ID NO5
c) 5'-CAG CCA ATT AGT ACT ATG AAA AGC-3'(SEQ ID NO6)

5. A purified 205y lipase without signal peptide and transmembrane domain is obtained from the preceding method claim 3 or 4, wherein purified 205y lipase without signal peptide and transmembrane domain including the steps of:
a) harvesting recombinant *E.coli* by centrifugation at 10 rpm, for 10 min at 4°C;
b) obtaining pellet from step (a) and dissolving the pellet in tris-HCL buffer (10mM) at pH between 7-7.3;
c) performing sonication of the pellet and obtaining cell lysate;
d) mixing the cell lysate with Bio-Lyte ampholyte (40% w/v);
e) obtaining a mixture of cell lysate with ampholyte and loading the mixture of cell lysate with ampholyte into a protein separation chamber;
f) obtaining purified fractions of protein.

6. A method of characterizing purified 205y lipase without a signal peptide and transmembrane domain, wherein the method includes the steps of:
a) analysing temperature on lipase activity and stability;
b) analysing pH on lipase activity and stability using buffers, wherein the buffers comprises acetate buffer (pH 4.0-6.0), potassium phosphate buffer (pH 6.0-8.0), tris-HCl buffer (pH 8.0-9.0), glycine-NaOH buffer (pH 9.0-11.0) and Na₂HPO₄/NaOH buffer;
c) analysing organic solvents on lipase activity, wherein the organic solvents comprises DMSO, methanol, ethanol, acetonitrile, n-decane, hexadecane, n-butanol, toluene, p-xylene, 1-decanol and n-dodecane ;
d) analysing metal ion on lipase activity, wherein the metal ions includes K⁺, Mg²⁺ Ca²⁺ ,Na⁺, Mn²⁺, Fe²⁺, Cu²⁺ and Zn²⁺;
e) analysing surfactants on lipase activity; wherein the surfactants comprises Tween 20, Tween 40 Tween 60 Tween 80, Triton X-100, SDS and SLS;
f) analysing inhibitors on lipase activity; wherein the inhibitors comprises dithiothreitol (DTT), β-mercaptoethanol, phenylmethylsulfonyl fluoride (PMSF), ethylenediaminetetraacetic acid (EDTA) and pepstatin A;
g) analysing substrate specificity of the 205y lipase, wherein the substrate comprises triacylglycerols (triacetin, tributyrin, tricaprilyn, triolein) and natural oils (olive oil, soy bean oil, sun flower oil, rice bran oil, coconut oil and palm oil);
h) determining positional specificity of the 205y lipase.

7. The method of characterizing purified 205y lipase according to claim 6(a), wherein the method further comprising the steps of:
a) preparing a reaction mixture by mixing 100 µL of purified enzyme with 2.5 mL substrate [olive oil: phosphate buffer pH 7.0, 1:1, (v/v)] and 20 µL CaCl_{2;}
b) heating the reaction mixture from step (a) at temperature between 30 °C and 70°C for 30 mins;
c) agitating the reaction mixture from step (b) at 200 revolution per min(rpm);
d) measuring lipase activity;
e) allowing the reaction mixture from step (b) to pre-incubate at temperature between 37 °C and 60°C for 30 mins;
f) agitating the reaction mixture from step (e) at 200 (rpm) at 60°C; and
g) measuring lipase activity
h) The method of characterizing purified 205y lipase according to claim 12 (b), wherein the method further comprising the steps of;
i) preparing a reaction mixture by mixing 100 µL of purified enzyme with 2.5 mL substrate [olive oil: the buffers between pH 4 and 12, 1:1, (v/v)] and 20 µL CaCl_{2;}
j) heating the reaction mixture from step (a) at temperature between 30 °C and 70°C for at least 30 mins;
k) agitating the reaction mixture from step (b) at 200 revolution per min(rpm);
l) measuring lipase activity;
m) allowing the reaction mixture from step (b) to pre-incubate with buffers at temperature at 37 °C between pH 4and 12 for at least 30 mins;
n) agitating the reaction mixture from step (e) at 200 (rpm) at 60°C; and
o) measuring lipase activity

8. The method of characterizing purified 205y lipase according to claim 6(c), wherein the method further comprising the steps of:
a) mixing purified enzyme with individual organic solvents (3:1 v/v) for at least 30 mins for pre-incubation;
b) agitating the reaction mixture from step (a) at 200 (rpm); and
c) measuring lipase activity

9. The method of characterizing purified 205y lipase according to claim 6(d), wherein the method further comprising the steps of:
a) mixing purified enzyme with individual metal ions according to claim 12(d), wherein the concentration of the metal ions between (1 and 10mM) and the concentration of purified lipase is between 1mM;
b) obtaining a reaction mixture;
c) adding potassion phosphate buffer of pH 8 into the reaction mixture from step (b) and pre-incubating the reaction mixture for at least 30 mins at 37 °C;
d) agitating the reaction mixture from step (c) at 200 (rpm); and
e) measuring lipase activity

10. The method of characterizing purified 205y lipase according to claim 6(e), wherein the method further comprising the steps of:
a) mixing purified enzyme with individual surfactants according to claim 12(e), wherein the concentration of the surfactants between 1 mM and the concentration of purified lipase is between 1mM;
b) obtaining a reaction mixture;
c) pre-incubating the reaction mixture from step (a) for at least 30 mins at 37 °C;
d) agitating the reaction mixture from step (c) at 200 (rpm); and
e) measuring lipase activity

11. The method of characterizing purified 205y lipase according to claim 6(f), wherein the method further comprising the steps of:
a) mixing purified enzyme with individual surfactants according to claim 6(f), wherein the concentration of the surfactants (between 1 and 5mM) and the concentration of purified lipase is between 1mM;
b) obtaining a reaction mixture;
c) pre-incubating the reaction mixture from step (a) for at least 30 mins at 37 °C;
d) agitating the reaction mixture from step (c) at 200 (rpm); and
e) measuring lipase activity .

12. The method of characterizing purified 205y lipase according to claim 6(g), wherein the method further comprising the steps of:
a) mixing purified enzyme with individual surfactants according to claim 6(g), wherein the concentration of the surfactants between 2.5 mL and the concentration of purified lipase is between 100µL pH 8.0, 1:1, (v/v)] and 20 µL CaCl₂;
b) obtaining a reaction mixture;
c) pre-incubating the reaction mixture from step (a) for at least 30 mins at 37 °C;
d) agitating the reaction mixture from step (c) at 200 (rpm); and
e) measuring lipase activity

13. The method of characterizing purified 205y lipase according to claim 6(h), wherein the method further comprising the steps of:
a) incubating 100µLof purified lipase with 100mM triolein and 50mM of potassium phosphate buffer between pH 8.for at least 30 mins at 37 °C;
b) obtaining a reaction product of triolein from step (a) by means of extracting the reaction product with 5mL of n-hexane;
c) obtaining an extract from step step (b) and analysing the extract by using a chromatography;
d) mixing the extract with a mixture of chloroform, acetone and acetic acid and obtaining a silica gel and
e) obtaining spots of glycerides and fatty acids by exposing the silica gel from step (d) to iodine vapour.

14. A purified 205y lipase without a signal peptide and transmembrane domain having the means of providing an activity between 55 °C and 65 °C, and/or stability at pH 5 and 10, and/or stability with organic solvents such as DMSO, methanol, ethanol, acetonitrile, n-decane, hexadecane, and/or an activity in the presence of K⁺, Mg²⁺ and Ca²⁺, and/or an activity in the presence of Tween 20, Tween 40 Tween 60 Tween 80, Triton X-100 and SDS, and/or an activity in the presence hydrolyzing triglycerides and natural oils at position 1 and 3.
